# EUROPEAN PATENT APPLICATION

(11) **EP 2 163 642 A1**
(43) Date of publication of application: **17.03.2010**
(21) Application number: 08156995.6
(22) Date of filing: 08.08.2001
(51) Int. Cl.: C12P 21/04, C12N 5/071, C07H 21/00, A01N 43/04, C07K 14/52, C07K 14/475, C12N 15/85, A61K 38/18, A61K 38/19, C07K 14/50, C07K 14/495, C07K 14/49, C07K 14/515, C07K 14/575, C12N 9/12

(54) **Nucleic acid constructs, vascular cells transformed therewith, pharmaceutical compositions and methods utilizing same for inducing angiogenesis**

(30) Priority: 08.08.2000 US 223727 P
(62) Divisional of application: 01958342.6
(71) Applicant: M.G.V.S. Ltd., Nazareth Illit 17106 (IL)
(72) Inventor: Flugelman, Moshe Y., 34642 Haifa (IL); Gluzman, Zoya, 25167 Tal-El (IL); Preis, Meir, 34402 Haifa (IL); Koren, Belly, 20692 Yokneam Ilit (IL); Cohen, Tzafra, 34521 Haifa (IL); Tsaba, Adili, 34323 Haifa (IL); Weisz, Anat, 34679 Haifa (IL)
(74) Representative: Machtalère, Georges

(57) **Abstract**

The present invention concerns a pharmaceutical composition comprising as active ingredients VEGF and Angiopoietin-1 (ANG-1) and a pharmaceutically acceptable carrier.

Sequence Listing

<110> Flugelman, Moshe et al.

<120> NUCLEIC ACID CONSTRUCTS, VASCULAR CELLS TRANSFORMED THEREWITH,
PHARMACEUTICAL COMPOSITIONS AND METHODS UTILIZING SAME FOR INDUCING
ANGIOGENESIS

<130> 01/22291

<150> US 60/227,727

<151> 2000-08-08

<160> 6

<170> PatentIn version 3.1

<210> 1

<211> 27

<212> DNA

<213> Artificial

<220>

<223> synthetic oligonucleotide

<400> 1
agatcttcaa aaatctaaag gtcgaat 27

<210> 2

<211> 27

<212> DNA

<213> Artificial

<220>

<223>  synthetic oligonucleotide

<400>  2
agatctgctg gcagtacaat gacagtt 27

<210>  3

<211>  21

<212>  DNA

<213>  Artificial

<220>

<223>  synthetic oligonucleotide

<400>  3
gctggcagta caatgacagt t 21

<210>  4

<211>  21

<212>  DNA

<213>  Artificial

<220>

<223>  synthetic oligonucleotide

<400>  4
tcaaaaatct aaaggtcgaa t 21

<210>  5

<211>  21

<212>  DNA

<213>  Artificial

<220>

<223>  synthetic oligonucleotide

<400>  5
gctggcagta caatgacagt t 21

<210>  6

<211>  21

<212>  DNA

<213>  Artificial

<220>

<223>  synthetic oligonucleotide

<400>  6
tcaaaaatct aaaggtcgaa t 21

## Description

### FIELD AND BACKGROUND OF THE INVENTION

The present invention relates to nucleic acid constructs, vascular cells transformed therewith and methods of utilizing such transformed vascular cells for inducing angiogenesis. More particularly, the present invention relates to genetically transformed vascular cells expressing at least one angiogenic proliferating factor and at least one angiogenic maturation factor and methods of utilizing the genetically transformed cells for inducing the formation of new blood vessels in mammalian tissue.

### Atherosclerotic Obstructive Cardiovascular Disease

Diseases caused by atherosclerosis such as coronary artery, cerebrovascular and peripheral vascular diseases are the most common cause of morbidity and mortality in the Western hemisphere [20].

Despite considerable public and private efforts, atherosclerosis-related diseases are second to no other known disease in terms of the economic burden imposed thereby. The World Health Organization (WHO) anticipates that atherosclerosis-related diseases will be the leading cause of morbidity and mortality in the world by the year 2020.

Various invasive treatment methods such as bypass surgery and angioplasty are routinely utilized to treat atherosclerosis-related disorders. Although these treatment methods have led to prolonged longevity in individuals suffering from atherosclerosis, such methods are limited by their complexity and highly invasive nature.

In addition, these methods cannot be effectively utilized in all patients suffering from ischemic conditions generated from blockage of an arterial tree or blockage of bypass grafts, either synthetic, venous or arterial. At present, public and privately funded cardiovascular research facilities are investing considerable time and resources in efforts to uncover novel therapeutic methods which will be effective in treating individuals suffering from atherosclerosis.

### Angiogenesis

In an adult, formation of new blood vessels in normal or diseased tissues is regulated by two processes, recapitulated arteriogenesis (the transformation of pre-existing arterioles into small muscular arteries) and angiogenesis, the sprouting of existing blood vessels (which occurs both in the embryo and in the adult) [1-5]. In the adult, physiological angiogenesis occurs during the female hormonal cycle, and in many pathological conditions, such as the formation of new blood vessels in and around a growing tumor and the formation of collateral vessels in an ischemic heart or limb [4,5].

The process of angiogenesis is regulated by biomechanical and biochemical stimuli. The angiogenic process consists of three sequential stages. In the first stage, termed initiation, the connection between endothelial cells and the surrounding tissue is severed. In the second stage EC proliferate and invade the ischemic tissue, which results in formation of EC sprouts. In the final stage of the angiogenic process the newly formed EC sprout matures into a functional blood vessel. The maturation process is characterized by recruitment of cells that surround the endothelial cells. Peri-endothelial cells, such as pericytes in the capillaries, smooth muscle cells in larger vessels and cardiac myocytes in the heart are recruited to provide maintenance and modulatory functions to the forming vessel.

The establishment and remodeling of blood vessels is controlled by paracrine signals, many of which are mediated by protein ligands which modulate the activity of transmembrane tyrosine kinase receptors [1,3,6]. Among these molecules are vascular endothelial growth factor (VEGF) and its receptor families (VEGFR1 and VEGFR2), Angiopoietin 1 and 2 (Ang-1 and Ang-2) and their receptors (Tie 2), basic fibroblast growth factor (bFGF), platelet derived growth factor (PDGF), and transforming growth factor β (TGF-β).

The dominant role of VEGF and its receptors in preliminary stages of angiogenesis and vasculogenesis has been clearly demonstrated in VEGF receptor null heterozygous animals [7-9]. Such animals, which do not survive the early stages of embryogenesis, either do not produce endothelial cells when heterozygous for the VEGFR1 receptor, or fail to form vessels when heterozygous for the VEGFR2 receptor. Studies which disrupted the Tie2 receptor or its ligands angiopoietin 1 and 2 demonstrated that although endothelial cells formed a tube, periendothelial cells were not recruited [10-14]. Interestingly, a similar phenotype was observed in animals lacking PDGF-B, TGF-β and tissue factor [15-18], suggesting that the binding of angiopoietin to its receptor may lead to the secretion of these factors from the endothelium. Recent studies have suggested that VEGF is responsible for the early stage of angiogenesis characterized by disintegration of the endothelial cells and leakage of plasma components [19,20]. These same studies also suggested that Ang-1 regulates the maturation of newly formed blood vessel, while other studies suggest that the binding of Ang-2 to Tie2 plays a role in the regression of already existing vessels [6,13].

The rate of angiogenesis involves a change in the local equilibrium between positive and negative angiogenic factors effecting the growth of microvessels. Partial elucidation of the role of angiogenic factors and their receptors has led to several proposed gene therapy procedures. Gene therapy is thought to be advantageous in vascular therapy, since a transgene can be expressed locally with no systemic side effects, its therapeutic effect can be pronounced over a prolonged period of time, and the effect can closely imitate normal biochemical regulation of vascular tissue [21-24].

Gene therapy was tested both *in-vitro* and in animal models for inhibition of smooth muscle cell proliferation following angioplasty and bypass surgery, and for induction of angiogenesis by enhancement of endothelial cell proliferation [23]. In addition, naked-DNA and recombinant adenoviral vectors encoding VEGF₁₆₅ and VEGF₁₂₁, respectively, were used for *in-vivo* gene transfer to genetically modify vascular cells of patients with ischemic and peripheral vascular disease [25,26]. However, the safety and efficiency of such therapeutic techniques was questioned following the death of 6 patients participating in these studies.

Although, it was demonstrated that co-administration of VEGF and Ang-1 encoding vectors in an animal model enhanced the development of collateral vessels [39], such direct administration of vectors would be of limited therapeutic effect in ischemic tissues and damaged organs, since a lack of healthy cells in such tissues limits expression levels.

Thus, due to the limitations described hereinabove, presently proposed or utilized treatment methods cannot be effectively or safely used to treat a wide range of individuals suffering from conditions generated by occlusion of peripheral, coronary and cerebrovascular blood vessels.

There is thus a widely recognized need for, and it would be highly advantageous to have, an effective, safe and mildly invasive method for inducing angiogenesis in a tissue region of an individual devoid of the above limitations.

### SUMMARY OF THE INVENTION

According to one aspect of the present invention there is provided a nucleic acid expression construct including: (a) a first polynucleotide segment encoding an angiogenic proliferating factor; and (b) a second polynucleotide segment encoding an angiogenic maturation factor.

According to another aspect of the present invention there is provided a nucleic acid expression construct system comprising (a) a first nucleic acid expression construct including a first polynucleotide segment encoding an angiogenic proliferating factor; and (b) a second nucleic acid expression construct including a second polynucleotide segment encoding an angiogenic maturation factor.

According to further features in preferred embodiments of the invention described below, the nucleic acid expression construct further including at least one promoter sequence being for directing the expression of at least one of the first and the second polynucleotide segments.

According to still further features in the described preferred embodiments the first polynucleotide segment is transcriptionally linked to the second polynucleotide segment whereas the first and the second polynucleotide segment are under the transcriptional control of a single promoter sequence of the at least one promoter sequence.

According to still further features in the described preferred embodiments the nucleic acid construct further including a linker sequence being interposed between the first and the second polynucleotide segments.

According to still further features in the described preferred embodiments the linker sequence is selected from the group consisting of IRES and a protease cleavage recognition site.

According to still further features in the described preferred embodiments the at least one promoter is functional in eukaryotic cells.

According to still further features in the described preferred embodiments the at least one promoter is selected from the group consisting of a constitutive promoter, an inducible promoter and a tissue specific promoter.

According to still further features in the described preferred embodiments the nucleic acid expression construct further including: (c) a first promoter sequence being for directing the expression of the first polynucleotide segment; and (d) a second promoter sequence being for directing the expression of the second polynucleotide segment.

According to still further features in the described preferred embodiments the first promoter and the second promoter are each selected from the group consisting of a constitutive promoter, an inducible promoter and a tissue specific promoter.

According to still further features in the described preferred embodiments expression from the first promoter and the second promoter is regulatable by one effector.

According to still further features in the described preferred embodiments the nucleic acid expression construct further including at least one additional polynucleotide segment encoding a marker polypeptide.

According to still further features in the described preferred embodiments the marker polypeptide is selected from the group consisting of a selection polypeptide and a reporter polypeptide.

According to still further features in the described preferred embodiments the at least one additional polynucleotide segment is transcriptionally linked to the at least one of the first and the second polynucleotide segments.

According to still further features in the described preferred embodiments the at least one additional polynucleotide segment is transcriptionally linked to the at least one of the first and the second polynucleotide segments via linker segment.

According to still further features in the described preferred embodiments the linker sequence is selected from the group consisting of IRES and a protease cleavage recognition site.

According to still further features in the described preferred embodiments the at least one additional polynucleotide segment is translationally fused to at least one of the first and the second polynucleotide segments.

According to still further features in the described preferred embodiments the angiogenic proliferating factor is selected from the group consisting of VEGF, acidic or basic FGF, PlGF, leptin and HGF.

According to still further features in the described preferred embodiments the angiogenic maturation factor is selected from the group consisting of Angiopoietin-1, Tie-2, TGF-β1, TGF-β receptor-2, endoglin, Smad5, VE-Cadherin, ephrinB2, PDGF, Bmx tyrosine kinase and MCP-1.

According to yet another aspect of the present invention there is provided a genetically transformed cell comprising the nucleic acid expression construct including: (a) a first polynucleotide segment encoding an angiogenic proliferating factor; and (b) a second polynucleotide segment encoding an angiogenic maturation factor.

According to still further features in the described preferred embodiments the transformed cell is selected from the group consisting of endothelial cells, smooth muscle cells, pericytes, myocytes, monocytes, and fibroblasts.

According to still further features in the described preferred embodiments the transformed cell is derived from a source selected from the group consisting of a segment of a vein or artery, bone marrow cells, peripheral blood progenitor cells, circulating endothelial cells and embryonic stem cells.

According to still further features in the described preferred embodiments the transformed cell is derived from a source selected from the group consisting of a human donor and an animal source.

According to still another aspect of the present invention there is provided a population of cells being genetically transformed to express at least one angiogenic proliferating factor and at least one angiogenic maturation factor.

According to still further features in the described preferred embodiments the population of cells includes at least two cell types selected from the group consisting of endothelial cells, smooth muscle cells, pericytes, myocytes, monocytes, fibroblasts and bone marrow derived cells.

According to still further features in the described preferred embodiments a first cell type of the at least two cell types is genetically transformed to express the at least one angiogenic proliferating growth factor and further wherein a second cell type of the at least two cell types is genetically transformed to express the at least one angiogenic maturation factor.

According to still further features in the described preferred embodiments the first cell type is an endothelial cell and further wherein the second cell type is a smooth muscle cell.

According to still further features in the described preferred embodiments the endothelial cell and the smooth muscle cell are each derived from a blood vessel segment.

According to still further features in the described preferred embodiments expression of each of the at least one angiogenic proliferating factor and the at least one angiogenic maturation factor is independently regulatable.

According to still further features in the described preferred embodiments the at least one angiogenic proliferating factor is selected from the group consisting of VEGF, acidic or basic FGF, P1GF, leptin and HGF.

According to still further features in the described preferred embodiments the at least one angiogenic maturation factor is selected from the group consisting of Angiopoietin-1, Tie-2, TGF-β1, TGF-β receptor-2, endoglin, Smad5, VE-Cadherin, ephrinB2, PDGF, Bmx tyrosine kinase and MCP-1.

According to an additional aspect of the present invention there is provided a method of inducing the formation of new blood vessels in a tissue region of an individual the method comprising the steps of: (a) administering a first cell type being genetically transformed to express at least one angiogenic proliferating factor into the tissue region of the mammal; and (b) administering a second cell type being genetically transformed to express at least one angiogenic maturation factor into the tissue region of the mammal.

According to still further features in the described preferred embodiments the tissue region includes occluded or narrowed segment of a blood vessel, ischemic muscle tissue or a bypass graft.

According to still further features in the described preferred embodiments the bypass graft is a synthetic graft or is derived from arterial or venous tissue.

According to still further features in the described preferred embodiments the first and the second cell types are derived from the individual.

According to still further features in the described preferred embodiments the first and the second cell types are each selected from the group consisting of endothelial cells, smooth muscle cells, pericytes myocytes, monocytes, fibroblasts and bone marrow stem cells.

According to still further features in the described preferred embodiments the method is utilized for bypassing or penetrating an occluded blood vessel segment or to establish collateral blood flow to an ischemic region.

According to still further features in the described preferred embodiments the first and the second cell types are co-administered into the tissue region of the individual.

According to still further features in the described preferred embodiments expression of the at least one angiogenic proliferating factor from the first cell type is regulatable by a first factor and further wherein expression of the at least one angiogenic maturation factor from the second cell type is regulatable by a second factor.

According to still further features in the described preferred embodiments the first and the second factors are a single factor capable of downregulating expression of the at least one angiogenic proliferating factor and upregulating expression of the at least one angiogenic maturation factor.

According to still further features in the described preferred embodiments step (b) is effected at least 12 hours following step (a).

According to still further features in the described preferred embodiments the first cell type and the second cell type are a single cell type.

According to still further features in the described preferred embodiments there is provided a pharmaceutical composition comprising, as an active ingredient, the nucleic acid constructs described hereinabove or the protein expressed thereby, and a pharmaceutically acceptable carrier.

The present invention successfully addresses the shortcomings of the presently known configurations by providing nucleic acid constructs, cells transformed therewith and methods of utilizing such transformed cells for inducing angiogenesis in mammalian tissues.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 illustrates a VEGF expression vector construct according to the teachings of the present invention.
FIG. 2 illustrates an Ang-1 expression vector construct according to the teachings of the present invention.
FIGs. 3a-b illustrate cultured EC transformed with VEGF-LacZ (3a) or VEGF-GFP (3b) expression constructs.
FIGs. 4a-c illustrates an occluded blood vessel (Figure 4a), a prior art bypass method for treating such an occlusion (Figure 4b) and methods of penetrating or bypassing the occluded blood vessel utilizing the teachings of the present invention (Figure 4c).
FIG. 5a illustrates sites of occlusion and flow measurement in a rat hind limb arterial tree artificially blocked and reopened by injecting a recombinant adenoviral viral vector expressing VEGF₁₆₅.
FIG. 5b is a graph illustrating blood flow in the rat femoral artery 3, 7, and 14 days following injection of the VEGF₁₆₅ adenoviral vector or saline (control). Values are presented as mean ±S.D., n=5 for each time point.
FIGs. 6a-d are light or fluorescence photomicrographs (Figures 6a-c and 6b-d, respectively) depicting EC or SMC (Figures 6a-b and 6c-d, respectively) infected with rAdAng1-GFP.
FIGs. 7a-d are light or fluorescence photomicrographs (Figures 7a-c and 7b-d, respectively) depicting EC or SMC (Figures 7a-b and 7c-d, respectively) transduced with retroAng1-GFP.

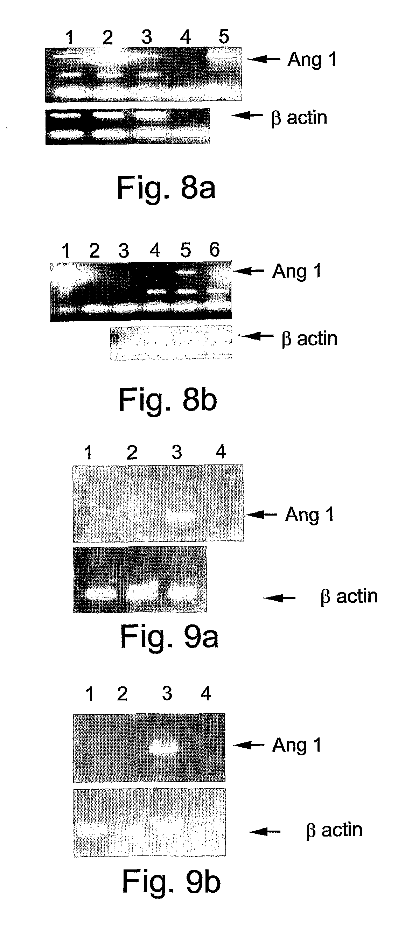

FIGs. 8a-b are photographs depicting expression of Ang-1 mRNA in EC and SMC (Figures 8a and 8b, respectively) 48 hours following adenoviral infection. Lanes in Figure 8a: 1-Control non-infected EC, 2-rAdAngl infected EC, 3-rAdGFP infected EC, 4-negative control of reverse transcription reaction, 5-positive control of plasmid Ang-1 DNA. Lanes in Figure 8b: 1-Ang-1 encoding plasmid DNA, 2-negative control of PCR reaction, 3-negative control of RT reaction, 4-rAdGFP infected SMC, 5-rAdAngl infected SMC, 6-Control non-infected SMC. Total RNA was extracted from EC and SMC following infection with adenoviral vectors and RT-PCR analysis was performed as described in the Examples section below. Samples were normalized by RT-PCR amplification of β-actin mRNA.
FIGs. 9a-b are photographs depicting expression of Ang-1 mRNA in EC and SMC (Figures 9a and 9b, respectively) 48 hours following retroviral transduction. Figure 9a lanes: 1-Control non-transduced EC, 2-retroGFP transduced EC, 3-retroAng1 transduced EC, 4-negative control of RT reaction, 5-positive control Ang-1 DNA encoding plasmid. Figure 9b lanes: 1-positive control Ang-1 DNA encoding plasmid, 2-negative control of PCR reaction, 3-negative control of reverse transcription reaction, 4- retroGFP transduced SMC, 5- retroAng1 transduced SMC, 6-control non-infected SMC. Total RNA was extracted from EC and SMC following transduction with retroviral vectors and RT-PCR analysis was performed as described in the Examples section below. Samples were normalized by RT-PCR amplification of β-actin mRNA.
FIG. 10 is a photograph depicting Ang-1 protein expression in adenoviral infected EC and SMC. Lanes: 1-control non-infected cells, 2-rAdGFP infected cells, 3-rAdUP50 infected cells, 4-rAdAngl infected cells, C-positive control Ang-1 recombinant protein.
FIG. 11 is a photograph depicting Ang-1 protein expression in retroviral transduced EC and SMC. Lanes: 1- non-transduced EC, 2-retroGFP transduced EC, 3-retroAngl transduced EC, 4-Non- transduced SMC, 5-retroGFP transduced SMC, 6-retroAngl transduced SMC, 7-Angl positive control.
FIGs. 12a-b are photographs depicting Western blot analysis of Ang-1 and VEGF₁₆₅ protein expression (Figures 12a and 12b, respectively) in co-cultures of adenovirally infected EC overexpressing Ang-1 with EC overexpressing VEGF₁₆₅. Lanes: 1-rAdVEGF infected EC + rAdAng1 infected EC, 2-rAdGFP infected EC + rAdAng1 infected EC, 3-rAdVEGF infected EC + rAdGFP infected EC, 4-positive control 293FLYA cells stably overexpressing Ang-1.
FIGs. 13a-b are photographs depicting Western blot analysis of VEGF₁₆₅ and Ang-1 protein expression (Figures 13a and 13b, respectively) in co-cultures of adenovirally infected EC overexpressing Ang-1 with SMC overexpressing VEGF₁₆₅. Lanes: 1-rAdVEGF infected EC + rAdAng1 infected SMC, 2-rAdGFP infected EC + rAdGFP infected SMC, 3-positive control 293FLYA cells stably overexpressing Ang-1.
FIG. 14 is a histogram depicting the normal proliferation of Ang-1-overexpressing rAdAng1-GFP infected EC. Cells were infected with rAdAng1-GFP (AdAng1), rAdVEGF-GFP (AdVEGF), rAdGFP (AdGFP) or non-infected (control). Results shown depict proliferation rate on Day 7 post-infection.
FIGs. 15a-b are photographs depicting enhanced phosphorylation of Tie2 receptor in rAdAng1 infected EC. Protein lysates of EC infected with rAdAng1-GFP, rAdGFP or rAdVEGF-GFP were analyzed for Tie2 phosphorylation by Western blot analysis without or with prior immunoprecipitation with anti-phosphotyrosine antibody (Figures 15a and 15b, respectively). Lanes: 1-non-infected EC, 2-rAdGFP infected EC, 3-rAdAng1-GFP infected EC, 4-rAdVEGF-GFP infected EC, 5-negative control 293 cells (Figure 15a only).
FIGs. 16a-h are photomicrographs depicting 3-dimensional *in vitro* angiogenic sprouting of collagen gel-embedded co-culture spheroids containing adenoviral infected EC. Panels depict rAdGFP infected EC (Figures 16a-b), co-cultured rAdGFP + rAdVEGF infected EC (Figures16c-d), co-cultured rAdAng1-GFP + rAdGFP infected EC (Figures 16e-f) and co-cultured rAdAng1-GFP + rAdVEGF-GFP infected EC (Figures 16g-h). Results from two representative 96-hour assays are shown.

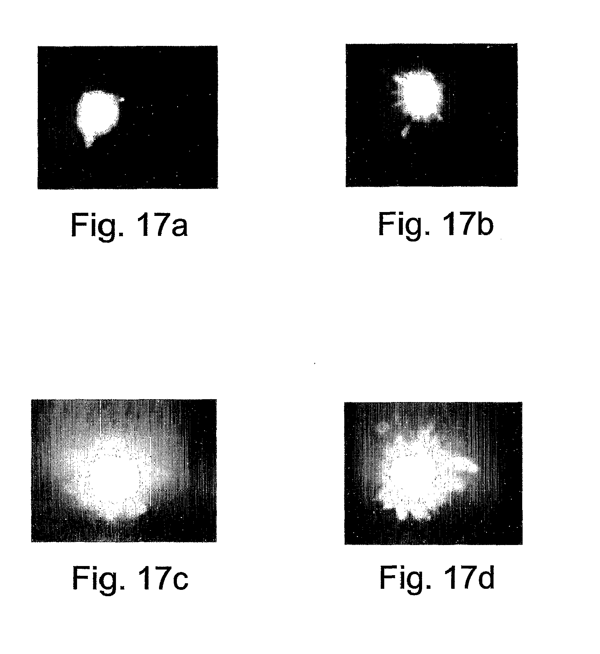

FIGs. 17a-d are fluorescence photomicrographs depicting 3-dimensional *in vitro* angiogenic sprouting of collagen gel-embedded co-culture spheroids containing adenoviral infected EC and retroviral transduced SMC. Panels depict co-cultured rAdGFP infected EC + retroGFP transduced SMC (Figure 17a), rAdVEGF-GFP infected EC + retroVEGF-GFP transduced SMC (Figure 17b), rAdGFP infected EC + retroAng1-GFP transduced SMC (Figure 17c), rAdVEGF-GFP infected EC + retroAng1-GFP transduced SMC (Figure 17d). Endothelial and SMC are indicated by red and green fluorescence, respectively. Representative 48-hour assays of each experimental group are shown.

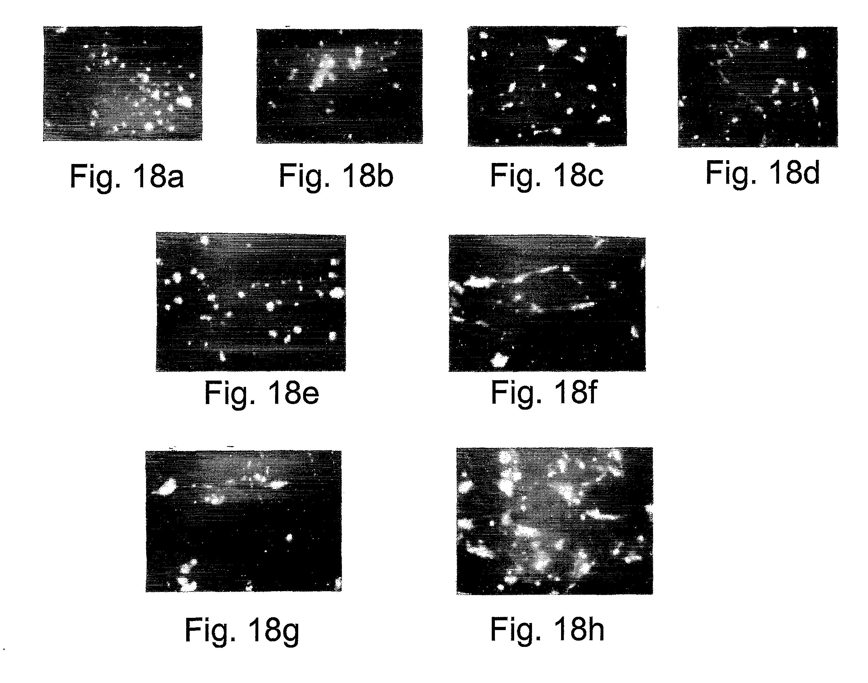

FIGs. 18a-h are fluorescence photomicrographs depicting survival of co-cultures of virally transformed vascular cells overexpressing angiogenic differentiation and proliferation factors under conditions of serum-starvation. Panels depict the following: rAdGFP infected SMC (Figure 18a), rAdAng1-GFP infected SMC (Figure 18b), rAdGFP infected EC (Figure 18c), rAdVEGF-GFP infected EC (Figure 18d), co-cultured rAdGFP infected SMC + rAdGFP infected EC (Figure 18e), co-cultured rAdGFP infected SMC + rAdVEGF-GFP infected EC (Figure 18f), co-cultured rAdAng1-GFP infected SMC + rAdGFP infected EC (Figure 18g) and co-cultured rAdAng1-GFP infected SMC + rAdVEGF-GFP infected EC (Figure 18h).
FIGs. 19a-h are light phase (Figures 19a, 19c, 19e and 19g) or fluorescence (Figures 19b, 19d, 19f and 19h) photomicrographs depicting enhanced survival of TUNEL-stained EC retrovirally transduced to overexpress Ang-1 following 24 hrs of serum deprivation. Shown are random fields representing non-transduced EC cultured with or without serum supplementation (Figures 19a-b and 19c-d, respectively) and retroAng1-GFP transduced EC cultured with or without serum supplementation (Figures 19e-f and 19g-h, respectively). Apoptotic cells are indicated by green fluorescence.
FIG. 20 is a histogram depicting percent apoptosis in retroviral transduced EC to overexpress Ang-1. Results presented represent quantification of the TUNEL assay data shown in the previous figure. Experimental groups consisted of: non-transduced EC, retroAng1-GFP transduced EC cultured with or without serum supplementation [serum(+) or serum(-), respectively]. Results shown as percent of apoptotic cells out of the total cell population counted in six random microscopic fields.

### DESCRIPTION OF THE PREFERRED EMBODIMENTS

The present invention is of nucleic acid constructs and vascular cells transformed therewith, which can be utilized for inducing angiogenesis. More specifically, the present invention can be utilized for inducing formation of new blood vessels capable of bypassing or penetrating occluded, injured or ischemic mammalian tissue.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

Prior art non-surgical methods for inducing and regulating angiogenesis have yet to become common practice in treatment of blood vessel blockage, injury or ischemia.

Such methods suffer from limitations inherent to the types of angiogenic factors used and/or to the modes of administration practiced.

To overcome the limitations inherent to prior art methods, the present inventors propose a novel method for promoting vascular tissue generation, which method is effected by providing two or more angiogenic factors including VEGF and Ang-1 to a site of treatment in an individual. Preferably, the angiogenic factors provided, are expressed and secreted from ex-vivo transformed endothelial and smooth muscle cells which are administered to the site of treatment.

Thus, according to one aspect of the present invention there is provided a nucleic acid expression construct including a first polynucleotide segment encoding an angiogenic proliferating factor such as but not limited to VEGF (GenBank Accession number AB021221), HGF (GenBank Accession number D14012), P1GF [28] (GenBank Accession number X54936), VEGF-C [30] (GenBank Accession number NM005429), bFGF [31] (GenBank Accession number J04513), aFGF (GenBank Accession number S67291) Leptin [32] (GenBank Accession number XM045426) or any other factor leading to endothelial cell proliferation and migration; and a second polynucleotide segment encoding an angiogenic maturation factor such as but not limited to Angiopoietin-1, TGF-β family (TGF-β1, TGF-β receptor-2, endoglin, Smad5) [33], VE-Cadherin [34], ephrinB2 [35], PDGF [36], Bmx tyrosine kinase [37], MCP-1 [38] or any other factor leading to blood vessel maturation and stabilization.

According to one preferred embodiment of this aspect of the present invention the angiogenic proliferating factor and the angiogenic maturation factor are expressed from a single promoter sequence included in the nucleic acid construct.

Various construct schemes can be utilized to effect co-expression of the first and second polynucleotide segments from a single promoter sequence.

For example, the first and second polynucleotide segments can be transcriptionally fused via a linker sequence including an internal ribosome entry site (IRES) sequence which enables the translation of the polynucleotide segment downstream of the IRES sequence. In this case, a transcribed polycistronic RNA molecule including the coding sequences of both the angiogenic proliferating factor and the angiogenic maturation factor will be translated from both the capped 5' end and the internal IRES sequence of the polycistronic RNA molecule to thereby produce both the angiogenic proliferating factor and the angiogenic maturation factor.

Alternatively, the first and second polynucleotide segments can be translationally fused via a protease recognition site cleavable by a protease expressed by the cell to be transformed with the nucleic acid construct. In this case, a chimeric polypeptide translated will be cleaved by the cell expressed protease to thereby generate both the angiogenic proliferating factor and the angiogenic maturation factor.

According to another preferred embodiment of this aspect of the present invention, the nucleic acid construct includes two identical or different promoter sequences such that the angiogenic proliferating factor and the angiogenic maturation factor are each separately transcribed from a dedicated promoter sequence.

It will be appreciated that expression of the angiogenic proliferating factor and the angiogenic maturation factor can be directed from two separate nucleic acid constructs.

Thus, according to another aspect of the present invention there provided a nucleic acid construct system. In this case, the first polynucleotide segment encoding an angiogenic proliferating factor is expressed from a first nucleic acid construct via a first promoter sequence, while the second polynucleotide segment encoding an angiogenic maturation factor is expressed from a second nucleic acid construct via a second promoter sequence which can be different or identical to the first promoter sequence.

As is further described hereinbelow, the nucleic acid constructs of the present invention are utilized for transforming mammalian cells such as, but not limited to, endothelial cells, smooth muscle cells, pericytes, myocytes, monocytes, fibroblasts, embryonic stem cells or bone marrow stem cells. As such, the promoter sequences utilized by the nucleic acid constructs of the present invention, are preferably constitutive, tissue specific or regulatable (e.g. inducible) promoters functional in such mammalian cell types.

To generate the nucleic acid constructs of the present invention, the polynucleotide segments encoding the angiogenic proliferating growth factor and/or the angiogenic maturation factor, can be ligated into a commercially available expression vector system suitable for transforming mammalian cells and for directing the expression of these factors within the transformed cells. It will be appreciated that such commercially available vector systems can easily be modified via commonly used recombinant techniques in order to replace, duplicate or mutate existing promoter or enhancer sequences and/or introduce any additional polynucleotide sequences such as, for example, sequences encoding additional selection markers or sequences encoding reporter polypeptides.

Suitable mammalian expression vectors for use with the present invention include, but are not limited to, pcDNA3, pcDNA3.1(+/-), pZeoSV2(+/-), pSecTag2, pDisplay, pEF/myc/cyto, pCMV/myc/cyto, pCR3.1, which are available from Invitrogen, pCI which is available from Promega, pBK-RSV and pBK-CMV which are available from Stratagene, pTRES which is available from Clontech, and their derivatives.

According to the teachings of the present invention, the angiogenic proliferating factor and the angiogenic maturation factor are provided to an ischemic tissue region in order to induce angiogenesis therein.

To avoid the safety issues associated with *in-vivo* gene transfer, and to enhance the formation of new blood vessels, the angiogenic factors are preferably provided from harvested, *ex-vivo* transformed, vascular cells.

Such transformed cells are utilized for inducing the formation of new blood vessels, by, for example, directly injecting the cells in or around the tissue region to be treated using a specially designed delivery catheters similar in principle to the perfusion catheters manufactured by Boston Scientific (USA).

Thus, according to another aspect of the present invention there is provided a population of cells genetically transformed to express at least one angiogenic proliferating factor and at least one angiogenic maturation factor.

Preferably, the cells are transformed ex-vivo, although in-vivo transformation of xenogeneic tissue followed by cell harvesting can also be utilized by the present invention.

As used herein the phrase "genetically transformed" refers to a cell transiently or stabely transformed with exogenous polynucleotide sequence(s). In stable transformation, the exogenous polynucleotide sequences integrate into the genome of the cell and as such are genetically inherited by daughter cells, whereas in transient transformation, the exogenous polynucleotide sequences exist in a transient manner as nuclear or cytoplasmic molecules and as such, are not genetically inherited by daughter cells.

The nucleic acid constructs of the present invention can be introduced into the population of cells via any standard mammalian transformation method. Such methods include, but are not limited to, direct DNA uptake techniques, and virus or liposome mediated transformation (for further detail see, for example, "Methods in Enzymology" Vol. 1-317, Academic Press).

According to this aspect of the present invention, the population of cells includes one or more cell types. Since, blood vessel generation and maturation is a stepwise process which involves several cell types expressing several angiogenic factors, the population of cells of the present invention preferably includes two distinct vascular cell types; a first cell type, such as, for example, an endothelial cell and a second cell type such as, for example, a smooth muscle cell, a pericyte cell or a myocyte cell.

Preferably, each cell type of the population of cells expresses a specific angiogenic factor. For example, the first cell type is transformed with a nucleic acid for expressing the angiogenic proliferating factor and the second cell type is transformed with a nucleic acid for expressing the angiogenic maturation factor.

It will be appreciated, however, that a population of cells in which the angiogenic proliferating factor and the angiogenic maturation factor are expressed from both cell types, or a population of cells in which one cell type expresses both factors while the other cell type expresses only one factor are also envisaged by the present invention.

As is further detailed hereinbelow, the population of cells according to the present invention is administered into a tissue region of an individual in order to bypass or penetrate, for example, an occlusion in a blood vessel supplying the tissue region. As is further described in the Examples section which follows, such administration can be directly into the occluded blood vessel or it can be into the tissue surrounding the occluded blood vessel.

As such, the cell types of the population of cells are preferably derived from venous or arterial tissue or bone marrow tissue of the individual to be treated or from tissue of a syngeneic individual. It will be appreciated that xenogeneic cells can also be utilized for preparing the population of cells providing measures are taken prior to, or during administration, so as to avoid rejection of such cells by the treated individual. Numerous methods for preventing or alleviating cell rejection are known in the art and as such no further detail is given herein.

According to another aspect of the present invention there is provided a method of inducing the formation of new blood vessels in a tissue region of an individual.

Since, the generation of new blood vessels depends on the timely provision of each cell type and more importantly of each angiogenic factor, the method of the present invention is preferably effected in a manner most suitable for generating such conditions in the tissue region to be treated.

Thus, according to one preferred embodiment of the present invention, angiogenesis is effected by administering a first cell type genetically transformed to express at least one angiogenic proliferating factor into the tissue region of the individual followed by administering a second cell type genetically transformed to express at least one angiogenic maturation factor into the tissue region of the individual.

The first cell type is preferably administered between 12 hours to 2 weeks prior to the administration of the second cell type. This ensures a timely provision of the angiogenic proliferation factor and the angiogenic maturation factor to the tissue to be treated thus ensuring optimal conditions for vessel formation.

It will be appreciated that a timely provision of the angiogenic factors can also be effected by administering one or more cell types which express the angiogenic factors from a promoter sequence regulatable by an effector.

As used herein the term "effector" or the phrase "regulatory factor" interchangeably refer to a molecule or a physical condition (e.g., light, biomechanical stress, etc.) capable of up-regulating or down-regulating the expression of a polynucleotide sequence from a promoter sequence regulated by such an effector. Examples of regulatable promoters which can be utilized by the present invention include chemically regulated promoters such as, for example, the tetracycline regulatable promoter system described in Agha-Mohammadi S, Lotze MT. Regulatable systems: applications in gene therapy and replicating viruses. J Clinical Investigations 2000;105:1177-1183, and biomechanical regulated promoters such as, a promoter including, for example, the shear stress responsive element described by Resnick et al., in PNAS USA 90:4591-4595, 1993.

As is further described in the Examples section which follows, one or more cell types can be transformed with a nucleic acid construct or constructs which express the angiogenic factors from regulatable promoter sequence(s). Such promoters are selected such that following administration of the cell type(s) into the tissue region to be treated, expression of the angiogenic factors can be upregulated or down regulated to produce the desired temporal expression pattern suitable for inducing the formation of new blood vessels.

Thus, for example, the expression of the angiogenic proliferating factor can be regulated by a first regulatory factor and the expression of the angiogenic maturation factor can be regulated by a second regulatory factor.

Such regulatory factors can be utilized at different time points following administration of the cells to thereby generate a different expression pattern for each of the angiogenic factors.

Alternatively, the promoter sequences can be selected such that the expression of the angiogenic maturation factor is upregulated by a factor, while the expression of the angiogenic proliferating factor is downregulated by the same factor. Thus, in this case a single regulatory factor can be utilized to generate a different expression pattern for each of the angiogenic factors.

It will be appreciated that the regulatable promoter is selected such that regulation thereof can be effected following administration of the cells into the tissue region to be treated. Thus, promoters which are regulatable by conditions generated during blood vessel formation, such as for example, forces associated with cell-to-cell interactions, or promoters which can be regulated by externally administered factors which can safely be provided to either the tissue region or the blood stream of the individual to be treated are preferred.

Thus, the present invention provides a novel approach for treating ischemic tissues. Enrichment of the ischemic organ with vascular cells genetically transformed to over express factors which improve cell survival while promoting blood vessel formation is a more rational and safe approach than prior art direct gene transfer approaches. The coupling of endothelial cells with smooth muscle cells and the expression of two different genes thereby ensures cooperation between the administered and recruited cells, to thereby ensure blood vessel formation and maintenance.

Although the angiogenic proliferating and maturation factors described hereinabove are preferably expressed from administered cells, such factors can also be administered as a polypeptide preparation which is either chemically synthesized or extracted from a transgenic prokaryotic or eukaryotic hosts expressing such factors.

Preferably the polypeptide preparation forms a part of a pharmaceutical composition. Such a pharmaceutical composition would also include a pharmaceutically acceptable carrier which serves to stabilize and enhnace targeting of the polypeptide factors.

Examples of suitable carriers include but are not limited to physiological solutions, liposomes, micelle bodies, viral particles and the like.

The pharmaceutical compositions of the present invention can be administered using any method known in the art. Preferably, the composition is injected in or around the tissue region to be treated as described hereinabove.

Thus, the present invention provides methods which can be used to promote the generation of new blood vessels or the recanalization of occluded or narrowed vascular tissue regions.

The present invention is substantially less invasive than bypass surgery or angioplasty and as such it traverses the risks associated with such surgical techniques.

Additional objects, advantages, and novel features of the present invention will become apparent to one ordinarily skilled in the art upon examination of the following examples, which are not intended to be limiting. Additionally, each of the various embodiments and aspects of the present invention as delineated hereinabove and as claimed in the claims section below finds experimental support in the following examples.

### EXAMPLES

Reference is now made to the following examples, which together with the above descriptions illustrate the invention in a non-limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996); all of which are incorporated by reference as if fully set forth herein. Other general references are provided throughout this document. The procedures therein are believed to be well known in the art and are provided for the convenience of the reader. All the information contained therein is incorporated herein by reference.

### EXAMPLE 1

### Expression vectors and virus particles

As described hereinabove, embodiments of the present invention utilize transformed vascular cells expressing VEGF and Ang-1 for inducing angiogenesis in ischemic, injured or occluded tissue. The Examples which follow outline methods for harvesting, conditioning and transforming vascular cells and methods of utilizing such cells in promoting angiogenesis.

### Materials and Methods:

### Preparation of retrovirus vector expressing the VEGF₁₆₅-GFP genes:

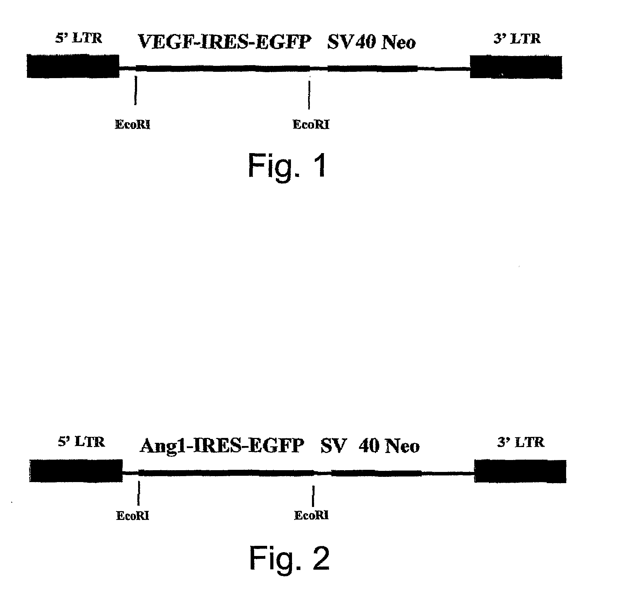

Figure 1 illustrates a VEGF-GFP expression vector construct according to the teachings of the present invention. The recombinant retroviral vectors expressing the human VEGF₁₆₅ (GenBank Accession number AB021221) and/or the EGFP (Clontech, Ca, USA) genes were constructed by separately cloning two different vectors into the LXSN plasmid (Clontech, Ca, USA). For construction of LXSN-based retroviral vector expressing GFP alone, an EcoRI-HpaI 1400 bp fragment containing an internal ribosome entry site (IRES) and the GFP gene was inserted into the specific restriction sites of the LXSN plasmid multiple cloning site (MCS). For construction of LXSN-VEGF-IRES-GFP bi-cistronic plasmid (Figure 1) which co-expresses the VEGF₁₆₅ and the GFP genes, a 2.0 kB EcoRI-MunI fragment encoding VEGF₁₆₅ (600 bp), and the IRES and EGFP sequences were ligated into the EcoRI restriction site of the LXSN MCS. Expression of transgenes by these LXSN vectors is regulated by the MoMULV long terminal repeat (LTR) (Clontech, Ca, USA). For retroviral vector production, 10 µg of LXSN-GFP or LXSN-VEGF-GFP plasmid DNA were transfected into 293E3 ecotropic packaging cells and incubated for 48 hours, following which supernatant from confluent cultures of G418 (Gibco BRL, USA) resistant producer cells was collected, filtered (0.45 µm) and added to PA317 amphotropic packaging cells. The transduced cells were exposed to G418 selection and culture supernatant was collected following 48 additional hours of culture and used to infect TEFLYGA amphotropic packaging cells which uniquely express the GALV envelope for high transduction efficiency of EC and resistance to human serum inactivation. Following G418 selection of transduced TEFLYGA cells, individual colonies were collected and screened for GFP and VEGF₁₆₅ expression. Viral titers of each colony were determined by TE671 cell transduction and titers ranging from 10⁵ to 10⁶ pfu/ml were obtained. Supernatant from the highest-titer producing colonies was collected and stored at -80°C.

The steps described above were applied to the construction of the Ang-1 expression vector construct LXSN-Ang1-IRES-EGFP (Figure 2), as described in Example 6.

***Preparation of packaging cell lines:*** Packaging cell lines were prepared according to known procedures. Detailed description of packaging cell lines can be found in the following references: Incorporation of fowl plague virus hemagglutinin into murine leukemia virus particles and analysis of the infectivity of the pseudotyped retroviruses. T. Hatziioannou, S. Valsesia-Wittmann, S.J. Russell and F.-L. Cosset. 1998. Journal of Virology, 72:5313-5317. Inverse targeting of retroviral vectors: selective gene transfer in a mixed population of hematopoietic and non-hematopoietic cells. A.K. Fielding, M. Maurice, F.J. Morling, F.-L. Cosset and S.J. Russell. 1998. Blood, 91:1802-1809. The fusogenic Gibbon ape leukemia virus envelope glycoprotein: targeting of a cytotoxic gene by ligand display. Fielding AK, Chapel-Fernandes S., Chadwick MP, Bullough FJ, Cosset F-L and Russell SJ. Human Gene Therapy, 2000;11;817-826*.*

### Transformed cell preparation:

***Autologous endothelial cell harvesting:*** EC are harvested from 5cm long human saphenous venin segments. Cells are harvested by collagenase digestion as previously described [27]. Cells from passages 3-9 are collected to ensure stability of cell characteristics. Cell identity is tested with immunohistochemistry for von Willebrand factor.

***Autologous smooth muscle cell harvesting:*** Smooth muscle cells were cultured by explant outgrowth from human saphenous veins (HSVSMC) and internal mammary arteries (HLSMC). Cells were cultured in Dulbecco's Modified Eagles Medium (DMEM) (Biological industries, Israel) supplemented with 10% pooled human serum (Beit Haemek, Israel). Smooth muscle cells were identified by using anti muscle actin antibodies (Zymed, USA).

***Bone marrow progenitor cell infection with VEGF encoding vectors:*** Bone marrow progenitor cells are infected with recombinant adenoviral vectors encoding VEGF-GFP. Differentiation is tested in infected cells using morphology and immunohistochemistry for von Willebrand factor. For more details regarding cell harvesting and in vitro differentiation related to neovascularization see: Asahara T. et al., EMBO J 1999;18:3964-72, and Huang XL, Takakura N, Suda T. Biochem Biophys Res Commun 1999, 264:133.

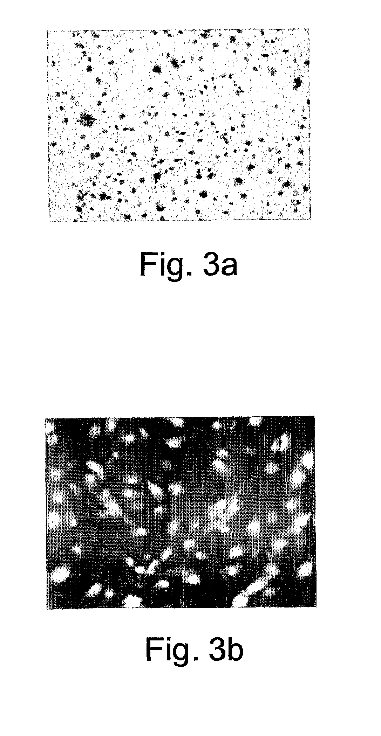

***Transduction of human and sheep saphenous vein EC by retroviral vectors:*** EC were seeded (10⁵ cells/35 mm dish) in fibronectin coated plates (Sigma, USA) and grown in M199 (Gibco-BRL, USA) containing 20% fetal serum. After 24 hours the cells were exposed to the cationic polymer DEAE-dextran (0.1 µg/ml) for 1 hour prior to transduction, washed 3 times with PBS and then transduced with a high titer of the recombinant virus vector encoding either the VEGF-LacZ or VEGF-GFP genes. Following incubation at 37 °C for 4 hours, virus-containing medium was replaced by fresh medium containing 20% pooled human serum. Figures 3a-b illustrate transgene expression from VEGF-LacZ or VEGF-GFP constructs, as indicated by LacZ activity or by GFP fluorescence, respectively.

***Regulation of gene expression after administration:*** An effector regulatable expression system is used in order to regulate gene expression of VEGF and Ang-1 in EC and/or SMC. For example, VEGF expression is downregulated via tetracycline, while Ang-1 expression is upregulated thereby. With these systems, the administered cells are made to express VEGF in the first week when cell proliferation is needed, while *in vivo* administration of tetracycline 12 hours to 2 weeks following *in vivo* implantation of cells downregulates VEGF expression and upregulates Ang-1 expression to promote cell maturation (Agha-Mohammadi S, Lotze MT. Regulatable systems: applications in gene therapy and replicating viruses. J Clinical Investigations 2000, 105:1177).

### EXAMPLE 2

### Transformed cell analysis

***Analysis of recombinant protein expression:*** VEGF and Ang-1 levels are measured in supernatant collected from cultures of genetically modified cells. ELISA and western blot analysis was used to measure VEGF or Ang-1 production over a 24 hour period. Following reinfusion of transformed cells *in vivo* into the donor, blood is collected and ELISA analysis of VEGF and Ang-1 levels is effected.

### EXAMPLE 3

### Animal experiments

***In Vivo Animal experiments:*** Short venous segment of experimental animals (derived from rabbit or sheep hind limbs) are used for endothelial cell harvesting. An alternative source for EC is bone marrow extracted via aspiration or circulating endothelial cell progenitors. Harvested cells are grown in the presence of VEGF to enhance cell differentiation and/or proliferation. Cells are expanded in the laboratory and genetically modified with pseudotyped retroviral vectors encoding VEGF and Ang-1.

The superficial femoral artery of each hind limb of the experimental animal is ligated immediately following bifurcation of the deep femoral artery. Blood flow is measured on both sides of the ligation using a Doppler flow meter (Transonic Animal Research Flowmeter, NY, USA). Following flow measurements, a population of homologous or heterologous vascular cells expressing VEGF and Ang-1 at various relative levels of expression are injected into one of the treated limbs, whereas a population of control cells expressing a marker polypeptide or PBS is injected into the second limb. Injection is performed proximally to the deep femoral artery. Flow is measured in both limbs 3, 7, 14 and 30 days following administration of the cells. Increased flow in the common femoral artery indicates the occurrence of angiogenesis.

***Evaluation of in vivo morphological changes:*** Following *in vivo* experiments, computerized analysis of digitized angiographic images obtained using contrast media are utilized to asses new blood vessel formation. Microscopic sections of relevant tissue are studied for the presence of implanted cells which are identified by marker polypeptide expression (e.g. GFP). The type and maturation stage of newly formed blood vessels is evaluated according to the morphology of such vessels (presence of cell types, number of elastic membranes, etc.)

### EXAMPLE 4

### Treatment approaches

### Materials and Methods:

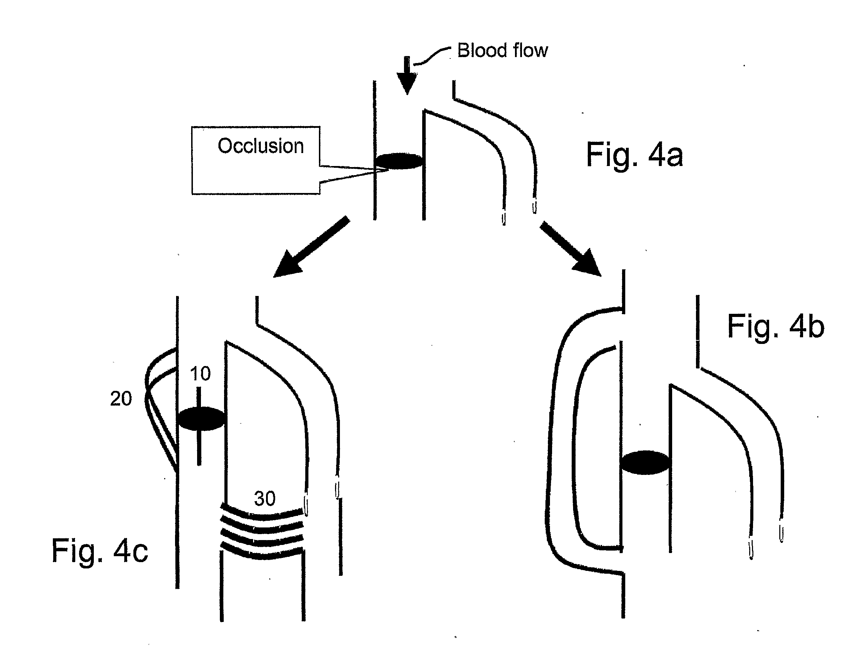

Three approaches can be utilized to treat an occluded blood vessel (Figure 4a). Figure 4b demonstrates prior art bypass surgery for treating a single occluded blood vessel. Figure 4c represent treatment alternatives for bypassing or penetrating the occluded blood vessel using the teachings of the present invention.

Figure 4c demonstrates angiogenesis in occluded tissue as a result of administration of vascular cells expressing at least one angiogenic proliferating factor and at least on angiogenic maturation factor into or around the occluded blood vessel. Such administration can lead to recanalization of the blood vessel, as indicated by **10,** or to the formation of a bypass around the occlusion, as indicated by **20.**

As specifically indicated by **30,** angiogenesis according to the teachings of the present invention can also lead to the formation of a vascular bridge which interconnects the occluded vessel to an unoccluded blood vessel thus effectively traversing the occlusion and reestablishing blood flow to the target tissue fed by the occluded blood vessel.

In any case, any of the above alternative methods can be utilized to restore blood supply to a tissue region fed by an occluded or injured blood vessel. In addition, the methods of the present invention can be utilized to restore blood supply even in cases of blood vessels which were previously treated via bypass surgery or angioplasty.

### EXAMPLE 5

### In vivo angiogenesis induced by ECgenetically modified to overexpress VEGF

The method of the present invention is designed to treat occlusive vascular disease by administration of EC genetically modified to induce optimal neovasculogenesis. Therefore, restoration of blood flow to occluded blood vessels via application of the method of the present invention was effected as follows.

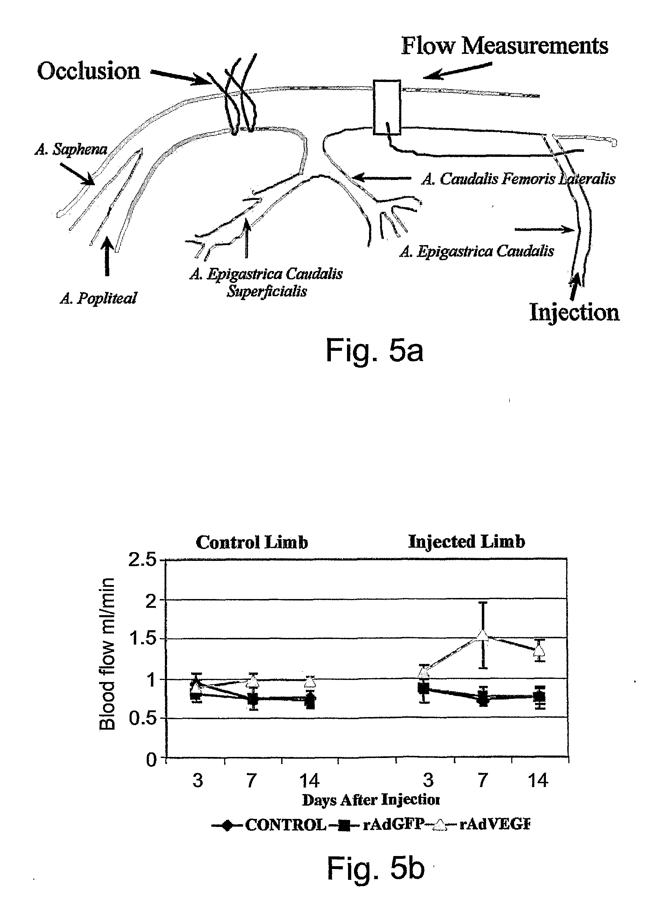

Male or female Sprague-Dolly rats weighing 400-450 g (Harlan, Israel) were anesthetized using ketamine and xylazine. The femoral arteries of these rats were surgically ligated distally to a large perforating artery. Rats were then injected with recombinant adenoviral vector encoding VEGF₁₆₅ (10⁹ pfu), recombinant adenoviral vector encoding GFP (10⁹ pfu) or normal saline. Injection was performed directly into a side branch of the femoral artery proximal to the ligated segment (Figure 5a). Blood flow in the femoral artery was measured at 3, 7, and 14 days following injection using a Doppler flow meter. Flow measured in rats treated with recombinant adenoviral vector expressing VEGF was significantly higher than that measured in rats injected with the recombinant adenoviral vector encoding GFP or saline (see Figure 5b). Localized expression of VEGF following injection was confirmed via RT-PCR of muscle tissue extracts and by measuring VEGF levels in blood plasma via ELISA (Data not shown).

These results, therefore demonstrate the effectiveness of the method of the present invention in treating occlusive vascular disease by inducing neovascularization of occluded blood vessels with EC genetically modified to overexpress pro-angiogenic factors. The genetically modified cells of the present invention can also be administered into an ischemic organ thus traversing the need for injecting potentially hazardous viral vectors into the blood stream, while enriching the ischemic organ with viable and activated vascular cells [40].

### EXAMPLE 6

### Expression of the angiogenic differentiation factor Ang-1 in EC and SMC genetically modified with viral vectors

Ideally, treatment of vascular diseases such as occlusive vascular diseases can be effected by formation of new blood vessels which bypass or alleviate vascular insufficiency. However, inducement of neovascularization processes *in vivo* using prior art techniques remains an unfulfilled challenge. The method of the present invention is designed so as to induce such neovascularization via the harnessing of multiple pro-angiogenic stimuli employed by vascular cells during natural growth of blood vessels. Such pro-angiogenic stimulation is conferred by the pro-angiogenic differentiation factor Ang-1 and the EC growth factor VEGF. Vascular cells expressing such factors thus represent an ideal therapeutic modality as these will both induce the differentiation of vascular tissues from precursor and their subsequent growth, thereby enabling the required therapeutic vessel formation.

Therefore, in order to effect expression of pro-angiogenic factors in vascular cells for treatment of vascular diseases of an occlusive nature, viral vectors for expression of Ang-1 and VEGF were constructed and expressed in EC and SMC as follows.

### Materials and Methods:

***Cloning of Ang-1 cDNA from human SMC:*** Human Ang-1 cDNA (nucleotide coordinates 310-1806 of Genbank accession number U83508) was reverse transcribed using AMV reverse transcriptase (RT) (Promega, USA) from total RNA extracted from human saphenous vein SMC. Briefly, 2 µg of RNA was mixed with 500 ng random hexamers, the mixture was heated at 70°C for 10 minutes and cooled on ice. To this preheated RNA was added a reaction mixture containing 0.5 mM dNTPs, 5 units AMV-RT, 5 mM DTT, 32 units RNAse out (Gibco-BRL, USA) and 1x Promega RT buffer. The resultant mixture was incubated at 38°C for 2 hours followed by 95°C for 15 minutes.

The cDNA product of the previous step was PCR-amplified using Expand High Fidelity PCR System (Roche, Switzerland).The following primers were employed for amplification: 5'-AGATCTTCAAAAATCTAAAGGTCGAAT-3' (SEQ ID NO:1) and 5'-AGATCTGCTGGCAGTACAATGACAGTT-3' (SEQ ID NO:2) (the underlined sequences represent the BglII restriction sites used for cloning). The PCR was performed in a volume of 20 µl containing 7 µl RT reaction mix cDNA product template, 20 pmole of each primer, 5nmole dNTPs, 1U Ex-Taq DNA Polymerase (Takara, Japan) and PCR reaction buffer provided by the manufacturer. The PCR reaction was performed as follows: 94°C/2' → 10×: [94°C/30" → 50°C/30" → 72°C/1'] → 24×: [94°C/30" → 56°C/30 → 72°C/(60" + 5"/cycle)] → 72°C/10'. The 1500 bp human Ang-1 cDNA (nucleotide coordinates 310-1806 of GenBank accession number U83508) was subcloned into pGEMT-easy vector (Promega). Both strands of the PCR product were sequenced and found to be 100% identical to the published sequence.

### Generation of recombinant viral vectors:

***Generation of recombinant adenoviral vectors encoding the Ang-1 gene:*** A recombinant adenoviral vector for expression of the human Ang-1 gene was constructed in several steps, consisting of routine prokaryotic cloning and a homologous recombination procedure in the 293-cell line. A 1500 bp fragment containing the human Ang-1 cDNA was inserted into pCA3 plasmid, under the control of the constitutive CMV immediate early promoter. The resultant plasmid was co-transfected with plasmid pJM17 into 293 cells which constitutively express the adenoviral E1 gene. Plasmid pJM17 contains the adenovirus genome, excluding the E3 region, and including an insert (pBRX) in the E1 region of the virus. Homologous recombination between pCA3 encoding Ang-1 and pJM17 following transfection replaced the E1 region of pJM17 with the CMV-Ang-1 expression cassette from pCA3. Plaque formation occurred 2 to 4 weeks following co-transfection after which individual plaques were isolated and viral extracts were amplified therefrom by infection of 293 cells. The titer of each viral stock was determined by plaque assay in 293 cells and viral titers of ∼10¹¹ pfu/ml were obtained.

Expression of Ang-1 protein in the growth medium of transformed cells was confirmed by Western blot analysis.

***Generation of recombinant adenoviral vectors encoding both the Ang-1 and GFP genes:*** Recombinant adenoviral vector rAdAng1-GFP encoding the human Ang-1 and GFP genes was constructed using a modified AdEasy protocol (He TC. et al., Proc Natl Acad Sci USA 1998, 95:2509). Briefly, the 1500 bp BglII fragment of human Ang-1 cDNA was inserted into the BglII site in pAdTrack-CMV shuttle vector for expression under the control of CMV promoter. This shuttle vector encodes the GFP gene downstream of the transgene under the control of a second CMV promoter. The Ang-1-GFP encoding shuttle vector was linearized by PmeI digestion and purified by Qiaquick Gel Extraction Kit (Qiagen, Germany). Competent BJ5183 cells were co-transformed with linearized Ang-1- and GFP-encoding shuttle vector and adenoviral vector pAdEasy-1 by electroporation. Transformants expressing recombinant adenoviral vector encoding Ang-1 and GFP genes were identified by PCR analysis and restriction mapping. Recombinant adenoviral plasmid vector was linearized by PacI digestion, purified and transfected into 293 cells using Lipofectamine 2000 (Gibco BRL, USA). Seven days following transfection cytopathic effect occurred; at this point, 100% of the cells expressed GFP. Cells were harvested, viral extracts were prepared and virus production was further amplified by infection of 293 cells with viral extract. The viral stock titers were determined by serial dilution assay in 293 cells and titers of ∼10¹¹ pfu/ml were obtained. Transgene expression was confirmed by Western blot analysis of infected cell-conditioned medium.

***Cloning of the Ang-1 gene in a retroviral expression vector:*** Recombinant retroviral vector pLXSN-Ang-1 expressing the human Ang-1 gene under the regulatory control of Mo-MULV 5' long terminal repeat (LTR) was constructed by cloning the 1500 bp human Ang-1 BglII cDNA fragment into the BamHI site of retroviral plasmid vector pLXSN (# K1060-B, Clontech, USA).

A recombinant bi-cistronic retroviral vector encoding both the human Ang-1 and EGFP genes under the regulatory control of Mo-MULV 5' long terminal repeat (LTR) was constructed by cloning these genes into pLXSN retroviral plasmid vector in a two-step process, as follows. First, an internal ribosomal entry site (IRES)-EGFP EcoRI-HpaI fragment (1400 bp) excised from pIRES2-EGFP (#6029-1 Clontech) was inserted into the EcoRI-HpaI sites of pLXSN for construction of the control plasmid pLXSN-IRES-EGFP. In a second step, pLXSN-Ang-1-IRES-EGFP was constructed by cloning the human Ang-1 1361 bp EcoRI fragment into the EcoRI site of pLXSN-IRES-EGFP.

***Generation of pseudotyped retroviruses for expression of the human Ang-1 gene:*** For retroviral vector production, 10 µg of pLXSN-IRES-EGFP, pLXSN-Ang-1-IRES-EGFP or pLXSN-Ang-1 retroviral vector plasmid was transfected into 293FLYA packaging cells using Lipofectamine (Gibco BRL, USA). After 48 hours supernatant from confluent cultures of viral producer cells was collected, 0.45 µm-filtered and added to 293FLY10A or 293FLYGALV amphotropic packaging cells. The transduced cells were subjected to G418 selection, individual colonies were collected and screened for EGFP expression by fluorescence microscopy and Ang-1 expression was confirmed by Western blot analysis of transduced cell-conditioned medium samples. Viral titers, determined by transduction of TE671 cells, were found to range from 10⁵ to 10⁶ pfu/ml. Supernatant from the colonies with the highest viral titers was collected and frozen at -80°C.

### Verification of transgene expression following gene transfer:

***Cell culture:*** Human saphenous vein EC (HSVEC) were isolated and cultured on gelatin-coated dishes in complete medium containing M199 medium (Gibco-BRL, USA) supplemented with 20% pooled human serum, 2 mM L-glutamine (Biological Industries, Israel), 100 units/ml penicillin, 0.1 mg/ml streptomycin (Biological Industries, Israel), 100 µg/ml heparin (Sigma, USA) and 2 ng/ml bFGF (kindly obtained from Prof. Neufeld, The Technion Institute, Haifa Israel). Human EC were identified by immunohistochemical analysis with anti von Willebrand factor antibodies (Zymed, USA). Smooth muscle cells were cultured by explant outgrowth from human saphenous vein SMC (HSVSMC) and human left internal mammary artery SMC (HLSMC). Cells were cultured in Dulbecco's Modified Eagles Medium (DMEM) (Gibco-BRL, USA) supplemented with 10% human serum, 2 mM L-glutamine, 100 units/ml penicillin, 0.1 mg/ml streptomycin and 2 ng/ml bFGF. Smooth muscle cells were identified by immunohistochemical analysis using anti-smooth muscle α-actin antibodies (Zymed, USA).

The packaging cell lines 293FLYA, 293FLY10A, 293FLYGALV and TEFLYGA (obtained from Dr F.L. Cosset, Lyon, France) were grown in DMEM supplemented with 10% FCS (Biologoical Industries, Israel), 2 mM L-glutamine, 100 units/ml penicillin, 0.1 mg/ml streptomycin, 6 µg/ml blasticidin (Sigma, USA) and 6 µg/ml phleomicin (Sigma, USA).

The packaging cell lines PA317, 293E3 (obtained from Dr. J. Exelrod, Jerusalem) were grown in DMEM supplemented with 10% FCS, 2 mM L-glutamine, 100 units/ml penicillin, and 0.1 mg/ml streptomycin.

***Infection of EC and SMC with recombinant adenoviral vectors:*** Endothelial cells and SMC were infected with recombinant adenoviral vectors as follows. Briefly, cells were seeded at ∼70% confluence in fibronectin (4.5 µg/ml)-pre-coated plates 20 hours prior to infection and cultured in complete medium (M20). On the day of infection the medium was replaced with fresh M199 (serum-free) medium and cells were infected with recombinant virus at 3000 viral particles/cell. The cells were incubated for 90 minutes with gentle tilting every 20 minutes after which the virus-containing medium was replaced with complete medium (M20). The infection rate was determined by visualization of GFP expression using an inverted fluorescence microscope (TE200 Nikon, Japan) equipped with a GFP fluorescence filter (GFP-LP, Nikon).

***Transduction of EC and SMC with recombinant retroviral vectors:*** Transduction of EC and SMC with retroviral vectors was performed as follows. Briefly, endothelial cells (passage 4-9) were seeded at 10⁵ cells/35 mm well in fibronectin (4.5 µg/ml)-coated plates and grown in complete medium for 24 h. One hour prior to transduction, the culture medium was replaced with serum-free M199 medium containing 0.1 mg/ml DEAE-dextran. Cells were washed three times with PBS and transduction was performed by incubating the cells for 4 hours with freshly collected and filtered (0.45 µm) supernatant from cultures of virus-producing packaging cells. At the end of the incubation the medium was replaced with fresh M20 medium.

### Ang-1 overexpression by infected EC and SMC:

***RT-PCR analysis of Ang-1 mRNA transcription:*** To detect Ang-1 gene transcription, total RNA was isolated from transduced HSVEC and HSVSMC using PURESCRIPT RNA Isolation Kit (Gentra systems, USA). For cDNA synthesis, 1 µg of RNA was mixed with 500 ng random hexamers heated at 70°C for 10 minutes after which the mixture was cooled on ice. A reaction mixture containing 0.4 mM dNTPs, 5 units AMV-RT (Promega, USA), 5mM DTT, 32 units RNAse-out (Gibco-BRL, USA) and 1x RT buffer (Promega, USA) was added to the preheated RNA-hexamer mix. Reverse-transcription was performed by incubating the reaction mixture at 38°C for 2 h followed by an incubation at 95°C for 15 min. For analysis of Ang-1 mRNA expression in HSVEC, PCR analysis of cDNA was performed in a volume of 20 µl using 7 µl of reverse transcribed cDNA product, 20 pmol sense primer (5'-GCTGGCAGTACAATGACAGTT-3', SEQ ID NO:3), 20 pmole anti-sense primer (5'-TCAAAAATCTAAAGGTCGAAT-3', SEQ ID NO:4), 5 nmol dNTPs, 1U Ex-Taq DNA polymerase (Takara, Japan) and PCR buffer provided by the manufacturer. The PCR was performed using the following set of incubations: 94°C/2' → 10x: [94°C/30" → 50°C/30" → 72°C/1'] → 24x: [94°C/30" → 56°C/30" → 72°C/(60" + 5"/cycle)] → 72°C/10'. For analysis of Ang-1 mRNA expression in HSVSMC, PCR analysis of cDNA was performed in a volume of 20 µl containing 7 µl of reverse transcribed cDNA product, 10 pmol sense and anti-sense primer (5'-GCTGGCAGTACAATGACAGTT-3', SEQ ID NO:5 and 5'-TCAAAAATCTAAAGGTCGAAT-3', SEQ ID NO:6, respectively), 5 nmol dNTPs, 1U Ex-Taq DNA polymerase (Takara, Japan) and PCR buffer provided by the manufacturer. The PCR was performed using the following set of incubations: 94°C/2' → 10×: [94°C/30" → 50°C/30" → 72°C/1'] → 21x: [94°C/30" → 60°C/30" → 72°C/(60" + 5"/cycle)] → 72°C/10'. PCR products were separated by electrophoresis in 0.8% agarose, labelled with EtBr and visualized by UV fluorescence.

***Western blot analysis:*** Detection of Ang-1 or VEGF₁₆₅ protein expression by adenovirally or retrovirally infected EC and SMC was performed by Western blot analysis of cell-conditioned medium 24 h post-infection, as follows. Briefly, virus-containing culture medium was replaced with serum-free medium and cells were grown for an additional 24 h. Secreted proteins in 30 µl samples of cell-conditioned medium were separated by PAGE in 10% SDS-polyacrylamide gel under reducing conditions and electroblotted onto nitrocellulose membrane (Schleicher & Schuell, Germany). Blots were blocked by incubation in TBS containing 0.1% skim milk and 0.3% Tween-20 (TBST) at room temperature for 1 hour with gentle agitation after which blots were incubated at room temperature for 2 hours with a 1:500 dilution of polyclonal goat anti-Ang-1 antibody (#sc-6319 Santa-Cruz, USA) or a 1:700 dilution of polyclonal rabbit anti-VEGF₁₆₅ antibody (#SC 152 Santa Cruz, USA) in blocking solution. Primary antibody-labelled blots were washed three times with TBST and incubated at room temperature for 1 hour with peroxidase-conjugated anti-rabbit or anti-goat secondary antibody (Sigma, USA) diluted in TBST according to the manufacturer's specifications. After three washes with TBST labelled protein was visualized by reacting blots with ECL reagents (Sigma, USA) and exposing these to X-ray film.

### Results:

***Viral constructs:*** The adenoviral and retroviral vectors constructed and employed to infect EC and SMC with Ang-1 are listed in Table 1.

**Table 1**

| Viral system | Gene(s) encoded | Virus designation |
|---|---|---|
| Adenovirus | GFP | rAdGFP |
| Adenovirus | Ang-1 | rAdAng1 |
| Adenovirus | Ang-1, GFP | rAdAng1-GFP |
| Adenovirus | VEGF₁₆₅, GFP | rAdVEGF-GFP |
| Pseudotyped Retrovirus | GFP | retroGFP |
| Pseudotyped Retrovirus | Ang-1 | retroAng1 |
| Pseudotyped Retrovirus | Ang-1, GFP | retroAng1-GFP |
| Pseudotyped Retrovirus | VEGF₁₆₅, GFP | retroVEGF-GFP |

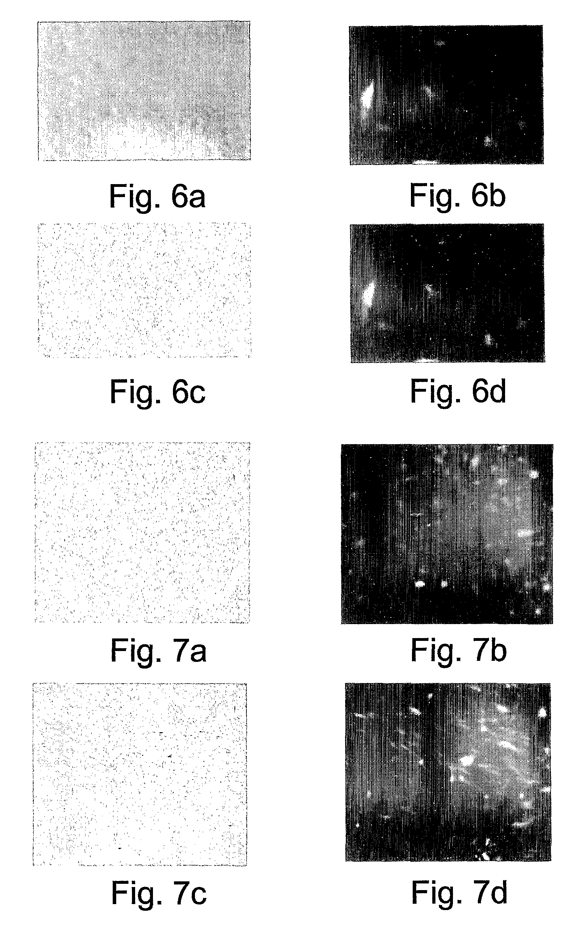

***Adenoviral infected or retroviral transduced EC and SMC express Ang-1-GFP transgene:*** Endothelial cells and SMC infected with rAdAng1-GFP were found to express high-levels of GFP (Figures 6b and 6d, respectively). Similarly, EC and SMC transduced with retroAng1-GFP were also found to express high-levels of GFP (Figures 7b and 7d, respectively). Expression of GFP was visualized by fluorescence microscopy.

Endothelial cells and SMC were found to transcribe Ang-1 mRNA 48 h following adenoviral infection with rAdAng1-GFP (Figures 8a and 8b, respectively). Similarly, EC and SMC retrovirally transduced with retroAng1-GFP were also found to express Ang-1 mRNA 48 h following transduction (Figures 9a and 9b, respectively).

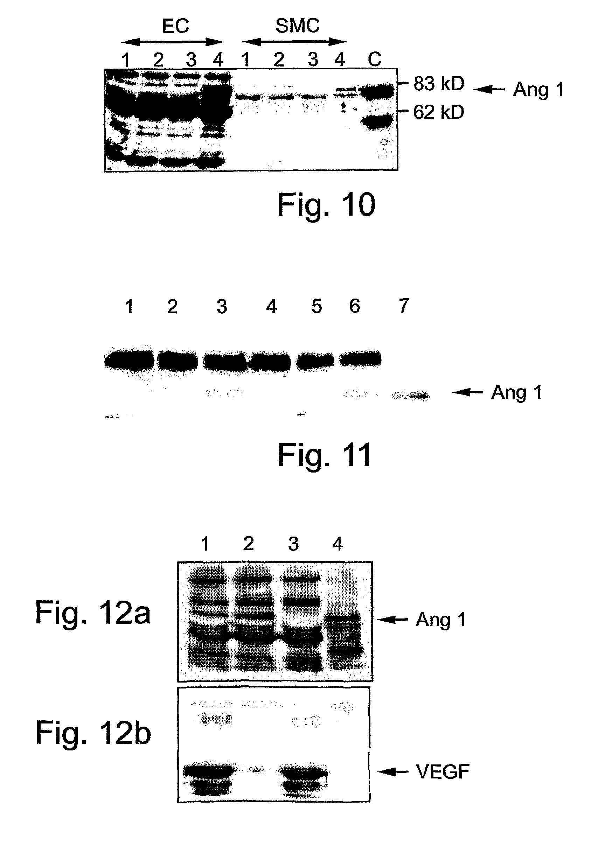

Endothelial cells and SMC were found to overexpress Ang-1 protein 24 h following adenoviral infection with rAdAng1 or following retroviral transduction with retroAng1 (Figures 10 and 11, respectively).

These results therefore demonstrate that the adenoviral and retroviral vectors of the present invention are competent to genetically modify primary human EC and SMC and to drive expression of Ang-1 and GFP transgenes therein. Hence, these constructs can be employed to genetically modify human vascular cells to overexpress the vascular maturation factor Ang-1 and thus may be effectively applied towards treatment of vascular diseases aimed at inducing the formation of new blood vessels capable of bypassing or penetrating occluded, injured or ischemic mammalian tissue.

### EXAMPLE 7

### Functional co-overexpression of Ang-1 and VEGF in vascular cells

In order to treat vascular disease, the method of the present invention employs administration of vascular cells co-overexpressing the angiogenic factors Ang-1 and VEGF. However, for such a therapeutic modality to be effective the overexpression of either one of these proteins must not interfere with either the expression or function of the other.

Thus, the ability of these proteins to be functionally co-overexpressed was demonstrated, as follows.

### Results:

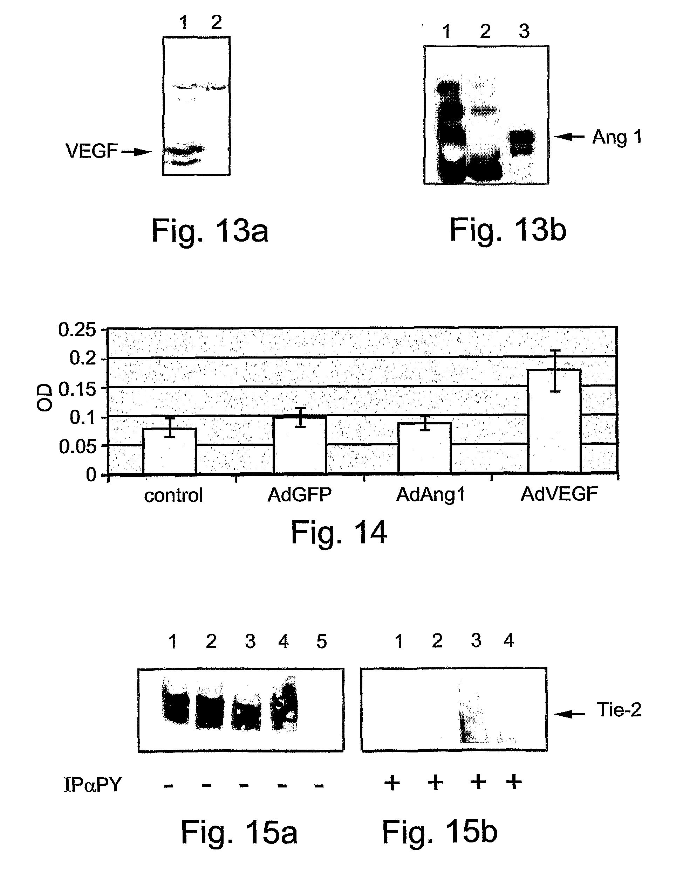

***Retention of Ang-1 and VEGF protein overexpression in co-cultures of adenovirally infected EC overexpressing Ang-1 and adenovirally infected EC or SMC overexpressing VEGF:*** The method of the present invention employs overexpression of both the vascular maturation factor Ang-1 and the angiogenic factor VEGF in genetically modified vascular cells, such as EC and SMC in order to optimize differentiation and growth of such cells so as to mediate optimal therapeutic effect when utilizing such cells for treatment of vascular disease. However, for such a therapeutic strategy to be optimally effective, the ability of EC overexpressing Ang-1 and of EC or SMC overexpressing VEGF to each retain overexpression of their respective transgene in the presence of overexpression of the other transgene is a requisite. Therefore retention of overexpression of both Ang-1 and VEGF protein in co-cultures of rAdVEGF infected EC and rAdAng1 infected EC was demonstrated via Western blot analysis (Figures 12a and 12b, respectively). Similarly, the concomitant overexpression of both VEGF and Ang-1 in co-cultures of rAdVEGF infected SMC and rAdAng1 infected EC was also demonstrated via Western blot analysis (Figures 13a and 13b, respectively).

### Overexpression of Ang-1 does not inhibit EC proliferation:

### Materials and Methods:

***Proliferation assays :*** Twenty-four hours prior to adenoviral infection, EC (passages 5-11) were seeded at 10⁴ cells/well (∼30% confluence) in 24-well plates pre-coated with fibronectin (4.5 mg/ml). Cells were infected with rAdAng1-GFP or rAdGFP and incubated at 37°C for 90 minutes after which serum-supplemented medium was added. After 16-18 hrs, the medium was substituted with M199 supplemented 2% human serum and 2 ng/ml bFGF. Proliferation assays were performed in triplicate and proliferation was assessed via XTT colorimetric assay on Days 3, 5, and 7 post-infection.

The method of the present invention includes therapeutic modalities for treatment of vascular disease in which Ang-1 and VEGF are co-overexpressed by vascular cells such as EC in the same milieu. However since the proliferative effect of VEGF on EC is preferably required for optimal effect, the absence of inhibitory effect of Ang-1 overexpression on the proliferation of rAdAng1-GFP infected EC was examined. Such overexpression of Ang-1 in EC was found to have neither inhibitory nor stimulatory effect on cellular proliferation when compared to the proliferation rate of cells infected with rAdGFP (Figure 14). As expected, proliferation of positive control infectants overexpressing VEGF was enhanced.

These results therefore demonstrate that coexpression of VEGF and Ang-1, according to the teachings of the present invention leads to the functional expression of both of these proteins. Hence, the method of the present invention, involving the co-overexpression of both of these proteins in genetically modified vascular cells can be effectively employed in the treatment of vascular diseases requiring neovascularization.

### EXAMPLE 8

### Biological activity of Ang-1 overexpression in vascular cells

The method of the present invention employs viral constructs for overexpression of Ang-1 in EC and includes modalities in which Ang-1 and VEGF are co-overexpressed to induce neovasculogenesis in the treatment of vascular diseases.

Thus, the functionality of the Ang-1 expressing adenoviral vectors of the present invention to drive expression of biologically active Ang-1 protein and the capacity of the method of the present invention to optimally induce neovasculogenesis via co-overexpression of Ang-1 and VEGF in vascular cells, such as EC and SMC, were demonstrated as follows.

### Materials and Methods:

***Immunoprecipitation-immunoblotting analysis of Tie2 phosphorylation in EC overexpressing Ang-1:*** Endothelial cells were seeded in 60 mm plates pre-coated with fibronectin (4.5 µg/ml) and infected with rAdAng1-GFP, rAdGFP, or rAdVEGF-GFP. Twenty-four hours post-infection, virus-containing medium was substituted with serum-free medium and the cells were cultured for an additional 24 hours prior to immunoprecipitation of cellular protein. Cells were washed in cold PBS supplemented with 100 µM Na₃VO₄ and lysed in lysis buffer containing 20 mM Tris-HCl (pH 7.5), 150 mM NaCl, 1% Triton X-100, 10% glycerol, 1 mM PMSF, 2 µg/ml leupeptin, 2 µg/ml aprotinin and 100 µM Na₃VO₄. Protein content of lysate supernatants was determined and lysate supernatant aliquots containing an equal quantity of protein were labelled with anti-phosphotyrosine antibody PY20 (Transduction Laboratories, USA) by incubation at 4°C overnight. Protein-A sepharose beads were added to the antibody-labelled samples and immunoprecipitation was performed by incubating the samples at 4°C for 2 h. The beads were subsequently washed 4 times in cold PBS, boiled in SDS-PAGE sample buffer for 3 min and pelleted by centrifugation. Supernatant proteins were separated electrophoretically in a 6% SDS-polyacrylamide gel. Separated proteins were electroblotted onto a nitrocellulose membrane (Schleicher and Schuell, Germany) and probed by Western immunoblotting analysis with anti-Tie2 antibodies (Santa Cruz, USA).

### Results:

***Biological activity of Ang-1: overexpression of Ang-1 in EC leads to enhanced Tie2 phosphorylation:*** Immunoprecipitation and immunoblotting analysis of Tie2 phosphorylation in EC adenovirally infected to overexpress Ang-1 demonstrated enhanced Tie2 phosphorylation (Figure 15b).

These results therefore demonstrate that the Ang-1 protein secreted by the constructs of the present invention are effective in binding to cognate receptor, Tie2, and hence that such constructs can be effectively employed to express the vascular maturation factor Ang-1 when treating vascular disease with genetically modified vascular cells, such as EC, according to the method of the present invention.

### In vitro angiogenesis assays - optimal three-dimensional angiogenic sprouting induced in co-cultured vascular cells overexpressing Ang-1 and VEGF:

The method of the present invention employs the use of both the vascular maturation and angiogenic factors Ang-1 and VEGF, respectively, in order to stimulate optimal angiogenesis for treatment of vascular diseases. Optimal angiogenesis by vascular cells, such as EC and SMC, overexpressing both of these factors was therefore demonstrated via analysis of three-dimensional sprouting of collagen gel-embedded co-culture spheroids containing virally transformed vascular cells as follows.

### Materials and Methods:

Sprouting (the in vitro equivalent of angiogenesis) by adenovirally infected spheroids of EC or by co-cultured spheroids of adenovirally infected EC and SMC was assessed in vitro by analyzing the sprouting of such spheroids in collagen gels, as previously described (Korff T. et al., J Cell Biol. 1998, 143:1341). Briefly, EC and SMC, pooled where applicable for generation of co-culture spheroids, were suspended in culture medium containing 0.25% (w/v) carboxymethylcellulose (Sigma, USA). For generation of EC-SMC co-cultures, EC were tagged with DiI-291 red fluorescent marker prior to mixing so as to enable differentiation of EC from SMC for subsequent fluorescence microscopic analysis of spheroids. Cell suspensions were seeded in non-adherent round-bottomed 96-well plates (Nunc, Denmark) so as to form a single spheroid of ∼750 cells per spheroid per well following 24 h of culture. The spheroids thus generated were then embedded in collagen gels, as follows. Collagen stock solution was prepared freshly prior to use by mixing 8 volumes of acidic extract of rat tail collagen (equilibrated to 2 mg/ml at 4°C) with 1 volume of 10x M199 medium (Gibco BRL, USA) and 1 volume of 0.34 N NaOH to adjust the pH to 7.4. Collagen stock solution was mixed at room temperature with an equal volume of M199 medium supplemented with 40% human serum containing 0.25% (w/v) carboxymethylcellulose to prevent spheroid sedimentation prior to polymerization of the gel. Gel samples containing 20-30 spheroids were rapidly transferred into pre-warmed 24-well plates and allowed to polymerize. The gels were incubated at 37°C, 5% CO₂ and the sprouting of at least 20 spheroids from each sample group was documented by digital video camera (DXM1200 Nikon, Japan).

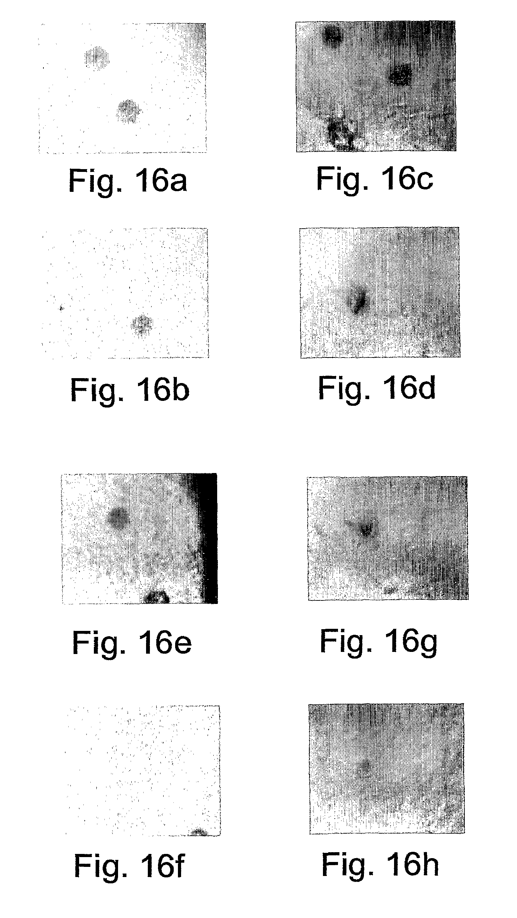

***Co-culture of Ang-1 and VEGF overexpressing vascular cells induces optimal angiogenesis:*** Spheroids of rAdGFP infected EC (Figure 16a-b) were observed, by fluorescent microscopic analysis, to display a low baseline sprouting activity, which was observed to be upregulated in co-culture spheroids containing rAdGFP infected and rAdVEGF-GFP infected EC (Figure 16c-d) as well as rAdAng1-GFP infected and rAdGFP infected EC (Figure 16e-f). The highest sprouting activity, however, was recorded in co-culture-spheroids containing rAdAng1-GFP and rAdVEGF-GFP infected EC (Figure 16g-h).

Similar results were obtained when co-culturing adenovirally infected EC and retrovirally transduced SMC. Co-culture spheroids containing rAdVEGF-GFP infected EC and retroGFP infected SMC were shown to moderately increase angiogenic sprouting activity relative to co-culture spheroids containing rAdGFP infected EC and retroGFP infected SMC (Figures 17b and 17a, respectively). Co-culture spheroids containing rAdGFP infected EC and retroAng1-GFP infected SMC displayed highly upregulated angiogenic sprouting activity (Figure 17c) however co-culture spheroids containing rAdVEGF-GFP infected EC and retroAng1-GFP infected SMC displayed synergistically increased levels of angiogenic sprouting activity (Figure 17d). These results were supported by the dramatically enhanced survival observed in co-cultures of rAdVEGF-GFP infected EC mixed with rAdAng1-GFP infected SMC cultured on glass slides for 24 hr under conditions of serum starvation (Figure 18h) as compared to rAdGFP infected SMC (Figure 18a), rAdAng1-GFP infected SMC (Figure 18b), rAdGFP infected EC (Figure 18c), rAdVEGF-GFP infected EC (Figure 18d), co-cultured rAdGFP infected SMC + rAdGFP infected EC (Figure 18e), co-cultured rAdGFP infected SMC + rAdVEGF-GFP infected EC (Figure 18f) and co-cultured rAdAng1-GFP infected SMC + rAdGFP infected EC (Figure 18g).

These results therefore demonstrate that by employing the co-overexpression of the vascular maturation and angiogenic factors Ang-1 and VEGF, respectively, in vascular cells, such as EC and SMC, the method of the present invention is capable of inducing optimal angiogenesis and cell survival. As such, the method of the present invention is superior to prior art techniques employing only one type of angiogenic factor for inducing neovascularization to treat vascular diseases.

### EXAMPLE 9

### Ang-1 overexpression mediates protection from apoptosis in genetically modified EC

The method of the present invention employs the genetic modification of vascular cells, such as EC, to overexpress the EC differentiation factor Ang-1 in conjunction with overexpression of the EC growth factor VEGF in order to induce optimal neovasculogenesis for treatment of vascular disease. However, in order to achieve such optimal therapeutic effect, it is highly desirable that such genetically modified cells overexpressing Ang-1 be capable to survive pro-apoptotic conditions which are likely to be encountered during all phases of treatment, ranging from the harvesting of primary cells, their genetic modification, their *ex vivo* culture and in particular upon reimplantation *in vivo* into ischemic tissues in which low oxygen tension and generally unfavorable conditions may reduce cell survival.

Thus, the capacity of EC retrovirally transduced to overexpress Ang-1 to display such resistance to pro-apoptotic conditions was demonstrated as follows.

### Materials and Methods:

***Terminal deoxynucleotide transferase (TdT)-mediated dUTP nick end labeling (TUNEL) assay of apoptotic cells:*** Briefly, non-infected EC or retroAng1-GFP transduced EC were seeded at 4 × 10⁵ cells/well in 24-well culture plates. Following 24 hr of culture, the cells were either further cultured for an additional 24 hr in M199 medium supplemented with 20% human serum or were cultured in serum-free M199 medium to induce apoptosis. Following exposure to pro-apoptotic conditions, all cells, floating and attached, were collected by trypsinization, fixed in freshly prepared PBS-4% formaldehyde, blocked with methanol-3% H₂O₂ and were further permeabilized in 0.1 % Triton X-100-0.1% sodium citrate. Finally, fragmented DNA ends were labeled by fluorescein-conjugated nucleotides with TdT and cells were examined by fluorescent microscopy by excitation at 450-500 nm and detection at 515-565 nm (green). The TUNEL assay was performed using In Situ Cell Death Detection Kit (cat # 1684817, Roche, Germany), according to the manufacturer's instructions.

### Results:

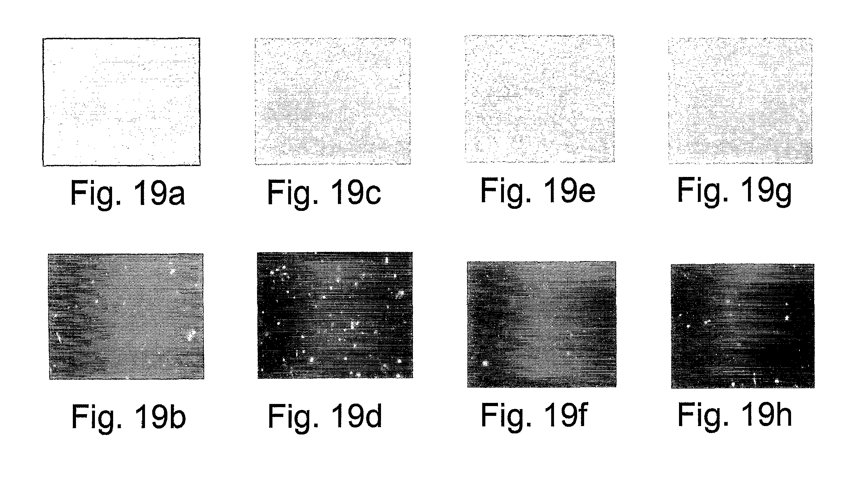

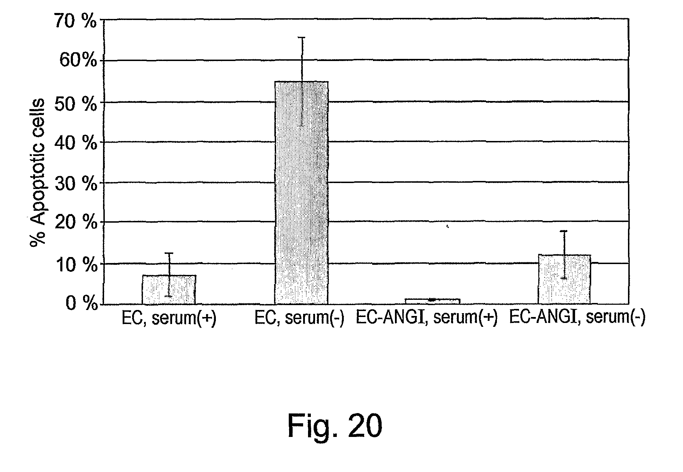

***Ang-1 overexpression mediates protection from apoptosis in genetically modified EC:*** Endothelial cells retrovirally transduced to overexpress Ang-1 display marked resistance to apoptosis under pro-apoptotic conditions as compared to non-transduced cells (Figures 19h and 19d), as visualized by microscopic fluorescent analysis of TUNEL stained cells. Numerical quantification performed on the data demonstrated microscopically above indicated that 55% of non-transduced EC were found to be apoptotic after 24 hrs of serum deprivation versus only 12% of EC retrovirally transduced to overexpress Ang-1 (Figure 20). Similar effects of reduced rate of apoptosis and improved survival were also observed in mixed populations of endothelial cells over expressing VEGF and Ang-1 (data not shown).

From these experiments it can therefore be concluded that overexpression of Ang-1 by vascular cells, preferably in conjunction with VEGF overexpression, optimizes the survival of such cells under physiological stresses likely to be encountered, for example, in ischemic organs. Thus, the present invention represents an improvement over prior art neovascularization methods to treat vascular diseases in general and in particular in pathologies involving ischemic tissues.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents, patent applications and sequences disclosed therein and/or identified by a GenBank accession number mentioned in this specification are herein incorporated in their entirety by reference into the specification, to the same extent as if each individual publication, patent, patent application or sequence was specifically and individually indicated to be incorporated herein by reference. In addition, citation or identification of any reference in this application shall not be construed as an admission that such reference is available as prior art to the present invention.

### REFERENCES

### (Additional references are cited in the text)

1. Korpelainen EI, Alitalo K. Signaling angiogenesis and lymphanogenesis. Current Opinion in Cell Biol 1998;10:159-164.
2. Rissau W. Mechanisms of angiogenesis. Nature 1997; 386:671-674.
3. Folkman J, D'Amore PA. Blood vessel formation: What is its molecular basis? Cell 1999;87:1153-56.
4. Schapper W, Ito WD. Molecular mechanisms of coronary collateral vessel growth. Circ. Res 1996; 79:911-19.
5. Ware JA, Simons M. Angiogenesis in ischemic heart disease. Nature Med 1997; 3:158-64.
6. Hanahan D. Signaling vascular morphogenesis and maintenance. Science 1997;277:48-50.
7. Ferrara N, Carver-Moore K, Chen H, et al. Embryonic lethality induced by targeted inactivation of the VEGF gene. Nature 1996;380:439-42.
8. Shalaby F, Rossant J, Yamaguchi TP, et al. Failure of blood islands formation and vasculogenesis in Flk-1 deficient mice. Nature 1995;376:62-66.
9. Fong GH, Rossant J, Gertenstein M, et al. Role of the Flt-1 receptor tyrosine kinase in regulating the assembly of vascular endothelium. Nature 1995;376:66-70.
10. Dumont DJ, Gradwhol G, Fong G-H, et al. Dominant-negative and targenull mutations in the endothelial receptor tyrosine kinase tek, reveal a critical role in vasculogenesis of the embryo. Genes Dev1994; 8:1897-1903.
11. Sato TN, Tzoawa Y, Deutsch U, et al. Distinct roles of the receptor tyrosine kinases Tie1 and Tie2 in blood vessel formation. Nature 1995;376:70-74.
12. Suri C, Jones PF, Patan S, et al. Requisite role of angiopoietin-1 a ligand for Tie2 receptor, during embryonic angiogenesis. Cell 1996;87:1171-80.
13. Maisonpierre PC, Suri C, Jones PF., et al. Angiopoietin-2 a natural antagonist for Tie2 that disrupts in-vivo angiogenesis. Science 1997;277:55-60.
14. Vikkula M, Boon LM, Carraway ML, et al. Vascular dysmorphogenesis caused by an activating mutation in the receptor tyrosine kinase Tie2. Cell 1996;87:1181-90.
15. Leveen P, Pekny M, Gebre-Medhin S, et al. Genes Dev 1994;8:1875-87.
16. Carmeliet P, Mackman N, Moons L, et al. Role of tissue factor in embryonic blood vessel development. Nature 1996;383:73-75.
17. Lindhal P, Johansson BR, Leveen P, Hbetsholtz C. Pericyte loss and microaneurysm formation in PDGF-B deficient mice. Science. 1997;277:242-245.
18. Leaky vessels? Call Ang1. Nat Med 2000;6:131-2.
19. Angiopoietin-1 protects the adult vasculature against plasma leakage. Nat Med 2000;6:460-3.
20. Braunwald E. Cardiovascular medicine at the turn of the millennium: triumphs, concerns, and opportunities. N Eng J Med 1997;337:1360-9.
21. Anderson WF, Human gene therapy. Science 256:808-813,1992.
22. Mulligan RC. The basic science of gene therapy. Science 1993;260:926-32.
23. Lewis BS, Flugelman MY, Weisz A, Keren-Tal I, Schaper W. Angiogenesis by gene therapy. Cardiovascular Research 1997; 35:490-7.
24. Kullo IJ, Simari RD, Schwartz RS. Vascular gene transfer. Arterioscler Thromb Vasc Biol 1999;19:196-207.
25. Isner JM. Pieczek A, Schainfeld R, et al. Clinical evidence of angiogenesis after arterial gene transfer of phVEGF165 in patient with ischemic limb. Lancet 1996;348:370-4.
26. Effect of intracoronary recombinant human vascular endothelial growth factor on myocardial perfusion: evidence for a dose-dependent effect. Circulation. 2000;101:118-21.
27. Flugelman MY, Virmani R, Leon MB, Bowman RL, Dichek DA. Genetically engineered endothelial cells remain adherent and viable after stent deployment and exposure to pulsatile flow. Circ Res 1992;70:348-354.
28. Dull RO, Yuan J, Chang YS, Tarbell J, Jain RK, Munn LL. Kinetics of placenta growth factor/vascular endothelial growth factor synergy in endothelial hydraulic conductivity and proliferation. Microvasc Res. 2001 Mar;61(2):203-10.
29. Salani D, Taraboletti G, Rosano L, Di Castro V, Borsotti P, Giavazzi R, Bagnato A. Endothelin-1 induces an angiogenic phenotype in cultured endothelial cells and stimulates neovascularization in vivo. Am J Pathol. 2000 Nov;157(5):1703-11.
30. Pepper MS, Mandriota SJ, Jeltsch M, Kumar V, Alitalo K.. Vascular endothelial growth factor (VEGF)-C synergizes with basic fibroblast growth factor and VEGF in the induction of angiogenesis in vitro and alters endothelial cell extracellular proteolytic activity. J Cell Physiol. 1998 Dec;77(3):439-52.
31. Asahara T, Bauters C, Zheng LP, Takeshita S, Bunting S, Ferrara N, Symes JF, Isner JM. Synergistic effect of vascular endothelial growth factor and basic fibroblast growth factor on angiogenesis in vivo. Circulation. 1995 Nov 1;92(9 Suppl):II365-71.
32. Cao R, Brakenhielm E, Wahlestedt C, Thyberg J, Cao Y. Leptin induces vascular permeability and synergistically stimulates angiogenesis with FGF-2 and VEGF. Proc Natl Acad Sci U S A. 2001 May 22;98(11):6390-5.
33. Pepper MS. Transforming growth factor β: Vasculogenesis, angiogenesis, and vessel wall integrity. Cytokine Growth Factor Rev. 1997;8:21-43.
34. Carmeliet P. et al. Targeted deficiency or cytosolic truncation of the VE-Cadherin gene in mice impairs VEGF-mediated endothelial survival and angiogenesis. Cell 1999;98:147-157.
35. Adams RH. et al. Roles of ephrinB ligands and EphB receptor in cardiovascular development: demarcation of arterial/venous domains, vascular morphogenesis, and sprouting angiogenesis. Genes & Development 1999;13:295-306.
36. Lindahl P. et al. Role of platlet-derived growth factors in angiogenesis and alveogenesis. Curr Top Pathol. 1999;93:27-33.
37. Rajantie I. et al. Bmx tyrosine kinase has redundant function downstream of angiopoietin and VEGF receptors in arterial endothelium. Molecular and cellular biology. 2001;21:4647-4655.
38. Ito WD. et al. Monocyte chemotactic protein-1increases collateral and peripheral conductance after femoral artery occlusion. Circ. Research 1997;80:829-837.
39. Chae JK, Kim I, Lim ST, Chung MJ, Kim WH, Kim HG, Ko JK, and Koh GY. Coadministration of Angiopoietin-1 and Vascular Endothelial Growth Factor Enhances Collateral Vascularization. Arterioscler. Thromb. Vasc. Biol. 2000; 20: 2573-2578.
40. Weisz A, Koren B, Cohen T, Neufeld G, Kleinberger T, Lewis BS, Flugelman MY. Increased vascular endothelial growth factor 165 binding to kinase insert domain-containing receptor after infection of human endothelial cells by recombinant adenovirus encoding the Vegf(165) gene. Circulation 2001 Apr 10;103(14):1887-92.

## Claims

1. A pharmaceutical composition comprising as active ingredients VEGF and Angiopoietin-1 and a pharmaceutically acceptable carrier.

2. Use of VEGF and Angiopoietin-1 for inducing blood vessel formation.

3. Use of VEGF and Angiopoietin-1 for treating a disease selected from the group consisting of atherosclerosis, ischemic disease and peripheral vascular disease.

4. An isolated nucleic acid expression construct comprising:
(a) a first polynucleotide segment encoding VEGF; and
(b) a second polynucleotide segment encoding ANG-1, and
wherein said VEGF and said ANG-1 are produced as separate polypeptides.

5. The isolated nucleic acid expression construct of claim 4, further comprising:
(c) a first promoter sequence being for directing the expression of said first polynucleotide segment; and
(d) a second promoter sequence being for directing the expression of said second polynucleotide segment.

6. A pharmaceutical composition comprising, as an active ingredient, the isolated nucleic acid expression construct of claim 4 and a pharmaceutically acceptable carrier.

7. Use of the nucleic acid expression construct of claim 4 for treating a disease selected from the group consisting of atherosclerosis, ischemic disease and peripheral vascular disease.

8. Use of a nucleic acid expression construct system comprising:
(a) a first nucleic acid expression construct including a first polynucleotide segment encoding VEGF; and
(b) a second nucleic acid expression construct including a second polynucleotide segment encoding ANG-1,
for treating a disease selected from the group consisting of atherosclerosis, ischemic disease and peripheral vascular disease.
